# EUROPEAN PATENT APPLICATION

(11) **EP 2 447 273 A1**
(43) Date of publication of application: **02.05.2012**
(21) Application number: 10792107.4
(22) Date of filing: 22.06.2010
(51) Int. Cl.: C07H 19/16, C07H 9/04

(54) **METHOD FOR SYNTHESIZING NUCLEIC ACID**

(30) Priority: 23.06.2009 JP 2009148994
(71) Applicant: Takeda Pharmaceutical Company Limited, Osaka-shi, Osaka 541-0045 (JP)
(72) Inventor: MURATA, Shumpei, Fujisawa Kanagawa 251-0012 (JP); UMEMOTO, Tadashi, Fujisawa Kanagawa 251-0012 (JP); MIYATA, Kenichi, Fujisawa Kanagawa 251-0012 (JP); HAYASE, Yoji, Fujisawa Kanagawa 251-0012 (JP)
(74) Representative: Held, Stephan
(86) International application number: PCT/JP2010/060572
(87) International publication number: WO 2010/150789

(57) **Abstract**

Provided is a new production method for the synthesis of an NC type nucleoside efficiently and conveniently in a high yield without unnecessary protecting group conversion steps. It relates to a step of producing a compound represented by the formula (II): 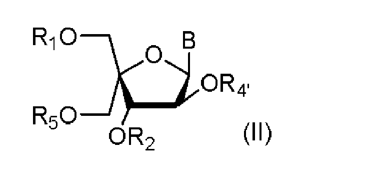 or a salt thereof, by inverting a compound represented by the formula (I): 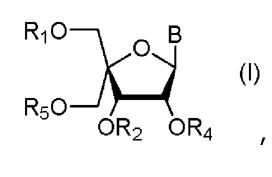 and
a method of producing a compound represented by the formula (III): 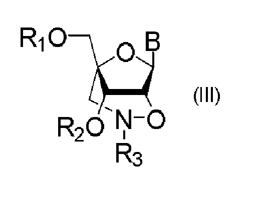 or a salt thereof (wherein each symbol is as defined in the specification), which includes the step.

## Description

### Technical Field

The present invention relates to a synthesis method of nucleic acid and an intermediate compound therefor. Particularly, the present invention relates to a production method of a compound which is an intermediate for producing an oligonucleotide analog having a superior antisense, antigene and RNA interference (RNAi) activity, and the intermediate compound.

### (Background of the Invention)

In 1978, an antisense oligonucleotide (antisense molecule) was first reported to have inhibited influenza virus infection. Thereafter, it has also been reported to have inhibited expression of cancer gene and AIDS infection. Since antisense oligonucleotide specifically regulates expression of undesirable genes, the field thereof is one of the most expected fields in recent years as a promising means for the development of pharmaceutical products.

However, when natural DNA or RNA oligonucleotide is applied as an antisense molecule to this method, many problems occur such as hydrolysis by enzymes in the body, not high cellular membrane permeability and the like. To solve such problems, nucleic acid derivatives have been synthesized, and the properties thereof have been studied. For example, phosphorothioates wherein an oxygen atom on the phosphorus atom of nucleic acid is substituted by a sulfur atom, and methylphosphonates wherein the oxygen atom is substituted by a methyl group have been synthesized, and recently, a molecule wherein a phosphorous atom is not contained in crosslinking element and a molecule wherein a ribose moiety is substituted by an acyclic skeleton have also been synthesized.

Patent document 1 reports that a DNA or RNA oligonucleotide containing an artificial nucleic acid 2',4'-BNA^{NC} unit represented by the formula

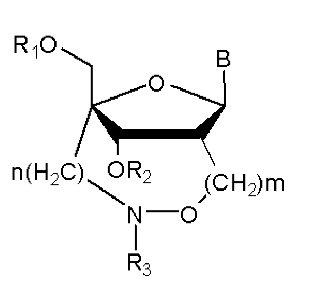

wherein each symbol is as defined in the document, and the carbon atom at the 2-position and the carbon atom at the 4-position of the sugar moiety in the nucleoside molecule are NO-bridged (in the present specification, such compounds are sometimes generically referred to as "NC type nucleoside") is a highly useful oligonucleotide derivative, since it shows a very high ability to form a double strand of complementary RNA chain, is superior in nuclease resistance, and permits binding of various other functional molecules to NO bond.

Patent document 2 reports that an oligonucleotide containing a nucleoside having 2'-O-CHR-4'crosslinking represented by the formula

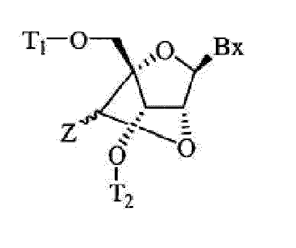

wherein each symbol is as defined in the document, is useful as a primer or probe, can be applied to a detection method of a nucleic acid molecule encoding a protein and the like, and a production method of said nucleotide. However, a production method of an NC type nucleoside wherein a carbon atom at the 2-position and a carbon atom at the 4-position are NO- bridged is not described.

Patent document 3 reports a production method of a bicyclonucleoside analog, which includes a step shown by the following formula. However, an inversion reaction at the 2-position is not described.

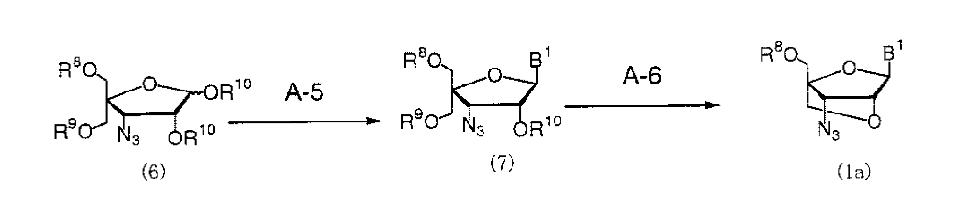

wherein each symbol is as defined in the document.

Patent document 4 reports a production method of oligonucleotide, which includes a step shown by the following formula. However, an inversion reaction at the 2-position is not described.

Method B

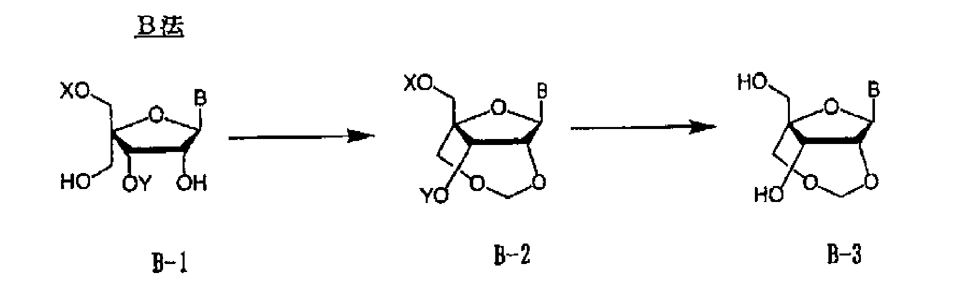

wherein each symbol is as defined in the document.

Non-patent document 1 describes a method of producing 9-(2-deoxy-2-fluoro-β-D-arabinofuranosyl)adenine via an inversion reaction. However, deprotection under oxidizing conditions does not proceed efficiently when the 2'-position is a benzyl group.

Non-patent document 2 describes a production method of 2'-ketonucleoside and 3'-ketonucleoside. However, the steric control of the reaction is based on steric hindrance of the protecting group, and uniform products cannot be obtained depending on the difference in the manner of protection and chemical modification of nucleoside derivatives.

Non-patent document 3 describes a production method of 2'-thioadenosine.
Non-patent document 4 describes a production method of RNA having not less than 100 nucleotide residues, which contains a nucleotide position-selectively substituted by a methylseleno group at the 2'-position. However, a production method of an NC type nucleoside wherein a carbon atom at the 2-position and a carbon atom at the 4-position are NO-bridged is not described.

There is still a demand for the development of a method of producing an NC type nucleoside, particularly a purine NC type nucleoside, efficiently and conveniently in a high yield.
Document List
patent documents

patent document 1: WO2005/021570
patent document 2: WO2007/090071
patent document 3: WO01/0745
patent document 4: WO03/068795
[non-patent documents]

non-patent document 1: The Journal of Organic Chemistry, 1992, 57, 553-559
non-patent document 2: Tetrahedron, Vol. 40, No. 1, pp. 125-135, 1984
non-patent document 3: Carbohydrate Research, 216(1991)257-269
non-patent document 4: Journal of the American Chemical Society, 2005, 127, 12035-12045

### Summary Of The Invention

### Problems to be Solved by the Invention

The present invention aims to provide a new production method for the synthesis of a purine NC type nucleoside efficiently and conveniently in a high yield.

### Means of Solving the Problems

The present inventors have conducted intensive studies in an attempt to solve the aforementioned problem and found that a compound represented by the formula (III) or a salt thereof (hereinafter sometimes to be abbreviated as compound (III)) can be produced efficiently and conveniently in a high yield without a conversion step of a protecting group, by inclusion of a step for inverting the steric chemistry of the 2'-position of a compound represented by the following formula (I) (hereinafter sometimes to be abbreviated as compound (I)) to give a compound represented by the formula (II) or a salt thereof (hereinafter sometimes to be abbreviated as compound (II)). They conducted further studies, which resulted in the completion of the present invention.

Accordingly, the present invention relates to the following:
[1] a method of producing a compound represented by the formula (II):

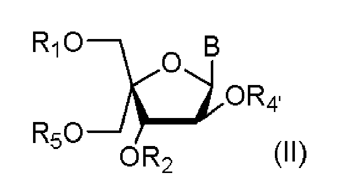

or a salt thereof, comprising inverting a compound represented by the formula (I):

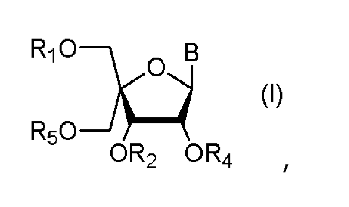

in each formula,
B is a purine ring group optionally having substituent(s);
R₁ is a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R₆)R₇
(R₆ and R₇ are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R₂ is a 2-naphthylmethyl group;
R₄ is an alkylsulfonyl group optionally substituted by a halogen atom;
R_{4'} is an acyl group optionally having substituent(s); and
R₅ is a sulfonyl group optionally having substituent(s),
[2] a method of producing a compound represented by the formula (III):

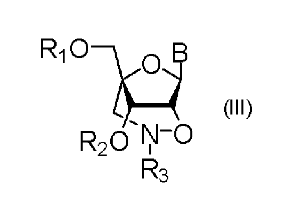

or a salt thereof, comprising a step of producing a compound represented by the formula (II):

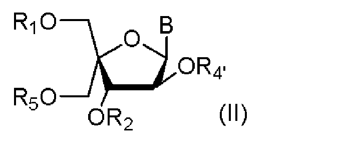

or a salt thereof, by inverting a compound represented by the formula (I):

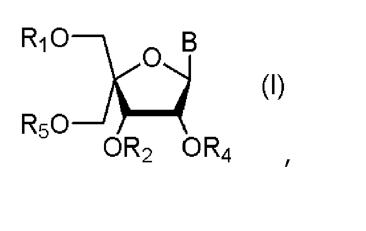

in each formula,
B is a purine ring group optionally having substituent(s);
R₁ is a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R₆)R₇
(R₆ and R₇ are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R₂ is a 2-naphthylmethyl group;
R₃ is a hydrogen atom, an alkyl group, an alkenyl group, a cycloalkyl group, an aryl group optionally having substituent(s), an aralkyl group optionally having substituent(s), an acyl group optionally having substituent(s), a sulfonyl group optionally having substituent(s), or a functional molecular unit substituent;
R₄ is an alkylsulfonyl group optionally substituted by a halogen atom;
R_{4'} is an acyl group optionally having substituent(s); and
R₅ is a sulfonyl group optionally having substituent(s),
[3] the method of the above-mentioned [1] or [2], wherein B is a purine ring group optionally having substituent(s), which is bonded at the 9-position of the purine ring,
[4] the method of the above-mentioned [1] or [2], wherein B is a purin-9-yl group optionally having substituent(s) selected from the following group α [group α: a hydroxyl group,
   a hydroxyl group protected by a protecting group for nucleic acid synthesis,
   an alkoxy group having a carbon number of 1 - 5,
   a mercapto group,
   a mercapto group protected by a protecting group for nucleic acid synthesis,
   an alkylthio group having a carbon number of 1 - 5,
   an amino group,
   an amino group protected by a protecting group for nucleic acid synthesis,
   an amino group substituted by an alkyl group having a carbon number of 1 - 5, an alkyl group having a carbon number of 1 - 5 and
   a halogen atom],
[5] the method of the above-mentioned [1] or [2], wherein B is
   a 6-aminopurin-9-yl group (i.e., adeninyl),
   a 6-aminopurin-9-yl group wherein the amino group is protected by a protecting group for nucleic acid synthesis,
   a 2,6-diaminopurin-9-yl group,
   a 2-amino-6-chloropurin-9-yl group,
   a 2-amino-6-chloropurin-9-yl group wherein the amino group is protected by a protecting group for nucleic acid synthesis,
   a 2-amino-6-fluoropurin-9-yl group,
   a 2-amino-6-fluoropurin-9-yl group wherein the amino group is protected by a protecting group for nucleic acid synthesis,
   a 2-amino-6-bromopurin-9-yl group,
   a 2-amino-6-bromopurin-9-yl group wherein the amino group is protected by a protecting group for nucleic acid synthesis,
   a 2-amino-6-hydroxypurin-9-yl group (i.e., guaninyl),
   a 2-amino-6-hydroxypurin-9-yl group wherein the amino group is protected by a protecting group for nucleic acid synthesis,
   a 6-amino-2-methoxypurin-9-yl group,
   a 6-amino-2-chloropurin-9-yl group,
   a 6-amino-2-fluoropurin-9-yl group,
   a 2,6-dimethoxypurin-9-yl group,
   a 2,6-dichloropurin-9-yl group or
   a 6-mercaptopurin-9-yl group,
[6] the method of the above-mentioned [1] or [2], wherein B is a 6-aminopurin-9-yl group (i.e., adeninyl),
[7] the method of any of the above-mentioned [1] to [6], wherein R₁ is
   (1) an aliphatic acyl group optionally having substituent(s),
   (2) an aromatic acyl group optionally having substituent(s),
   (3) an aliphatic sulfonyl group optionally having substituent(s),
   (4) an aromatic sulfonyl group optionally having substituent(s),
   (5) a methyl group substituted by 1 to 3 aryl groups optionally having 1 to 3 substituents, or
   (6) -Si(R₈)(R₉)(R₁₀)
      wherein R₈, R₉ and R₁₀ are the same or different and each is
      (i) a lower alkyl group or
      (ii) an aryl group optionally having substituent(s) selected from the group consisting of a lower alkyl group, a lower alkoxy group, a halogen atom and a cyano group,
[8] the method of any of the above-mentioned [1] to [6], wherein R₁ is
   an acetyl group,
   a benzoyl group,
   a methanesulfonyl group,
   a p-toluenesulfonyl group,
   a benzyl group,
   a p-methoxybenzyl group,
   a trityl group,
   a dimethoxytrityl group,
   a monomethoxytrityl group or
   a tert-butyldiphenylsilyl group,
[9] the method of any of the above-mentioned [1] to [6], wherein R₁ is a tert-butyldiphenylsilyl group,
[10] the method of any of the above-mentioned [2] to [9], wherein R₃ is
   a hydrogen atom,
   a phenoxyacetyl group,
   an alkyl group having a carbon number of 1 - 5,
   an alkenyl group having a carbon number of 2 - 5,
   an aryl group having a carbon number of 6 - 14 and optionally having substituent(s),
   a methyl group substituted by 1 to 3 aryl groups optionally having substituent(s), a lower aliphatic sulfonyl group optionally having substituent(s),
   an aromatic sulfonyl group optionally having substituent(s),
   an aliphatic acyl group having a carbon number of 1 - 5 and optionally having substituent(s), or
   an aromatic acyl group optionally having substituent(s),
[11] the method of any of the above-mentioned [2] to [9], wherein R₃ is
   a hydrogen atom,
   an alkyl group having a carbon number of 1 - 5,
   a p-toluenesulfonyl group,
   a methanesulfonyl group, or
   a trifluoromethanesulfonyl group,
[12] the method of any of the above-mentioned [2] to [9], wherein R₃ is an alkyl group having a carbon number of 1 - 5,
[13] the method of any of the above-mentioned [2] to [9], wherein the functional molecular unit substituent for R₃ is a fluorescence□luminescence-labeling molecule or chemical luminescence-labeling molecule, a functional group having nucleic acid cleavage activity, or an intracellular or nuclear localization signal peptide,
[14] the method of any of the above-mentioned [1] to [13], wherein R₄ is a lower alkylsulfonyl group optionally substituted by a halogen atom,
[15] the method of any of the above-mentioned [1] to [13], wherein R₄ is a trifluoromethanesulfonyl group,
[16] the method of any of the above-mentioned [1] to [15], wherein R_{4'} is an alkyl having a carbon number of 1 - 6 - carbonyl group optionally having substituent(s),
[17] the method of any of the above-mentioned [1] to [15], wherein R_{4'} is an acetyl group,
[18] the method of any of the above-mentioned [1] to [17], wherein R₅ is an aromatic sulfonyl group optionally having substituent(s),
[19] the method of any of the above-mentioned [1] to [17], wherein R₅ is a phenylsulfonyl group optionally having substituent(s),
[20] the method of any of the above-mentioned [1] to [17], wherein R₅ is a p-toluenesulfonyl group,
[21] the method of any of the above-mentioned [1] to [20], wherein the reaction is performed in a solvent in the presence of an organic acid salt of an alkali metal,
[22] the method of the above-mentioned [21], wherein the solvent is a sulfoxide solvent,
[23] the method of the above-mentioned [21], wherein the solvent is dimethyl sulfoxide,
[24] the method of any of the above-mentioned [21] to [23], wherein the organic acid salt of alkali metal is alkali metal acetate,
[25] the method of any of the above-mentioned [21] to [23], wherein the organic acid salt of alkali metal is cesium acetate,
[26] the method of any of the above-mentioned [21] to [25], wherein the reaction temperature is room temperature,
[27] a compound represented by the formula:

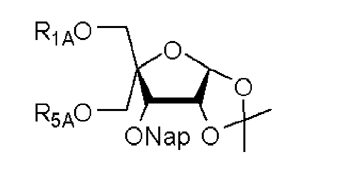

wherein
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
(R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R_{5A} is a sulfonyl group optionally having substituent(s); and
Nap is a 2-naphthylmethyl group;
or a salt thereof,
[28] the compound of the above-mentioned [27], wherein R_{1A} is
   -Si(R_{8A})(R_{9A})(R_{10A})
   wherein R_{8A}, R_{9A} and R_{10A} are the same or different and each is
   (i) a lower alkyl group, or
   (ii) an aryl group optionally having substituent(s) selected from the group consisting of a lower alkyl group, a lower alkoxy group, a halogen atom and a cyano group,
   or a salt thereof,
[29] a compound represented by the formula:

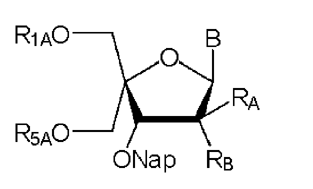

wherein
B is a purine ring group optionally having substituent(s);
one of R_{A} and R_{B} is a hydrogen atom, and the other of R_{A} and R_{B} is a hydroxyl group optionally having substituent(s);
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
(R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R_{5A} is a sulfonyl group optionally having substituent(s); and
Nap is a 2-naphthylmethyl group; and
R_{1A} is not 2-naphthylmethyl, or a salt thereof,
[30] the compound of the above-mentioned [29], wherein R_{1A} is -Si(R_{8A})(R_{9A})(R_{10A})
   wherein R_{8A}, R_{9A} and R_{10A} are the same or different and each is
   (i) a lower alkyl group, or
   (ii) an aryl group optionally having substituent(s) selected from the group consisting of a lower alkyl group, a lower alkoxy group, a halogen atom and a cyano group, or a salt thereof,
[31] the compound of the above-mentioned [29] or [30], wherein R_{A} is a hydrogen atom, and
   R_{B} is a hydroxyl group optionally having substituent(s), or a salt thereof,
[32] the compound of the above-mentioned [29] or [30], wherein R_{A} is a hydrogen atom, and
   R_{B} is a hydroxyl group optionally having substituent(s) selected from
   (1) an acyl group optionally having substituent(s);
   (2) a sulfonyl group optionally having substituent(s); and
   (3) -NR_{11A}R_{12A}
      (R_{11A} and R_{12A} are the same or different and each is
      (i) a hydrogen atom,
      (ii) a hydrocarbon group optionally having substituent(s),
      (iii) a heterocyclic group optionally having substituent(s),
      (iv) an acyl group optionally having substituent(s), or
      (v) a sulfonyl group optionally having substituent(s), or
   R_{11A} and R_{12A} form, together with the adjacent nitrogen atom, a cyclic group optionally having substituent(s)),
   or a salt thereof,
[33] the compound of the above-mentioned [29] or [30], wherein R_{A} is a hydroxyl group optionally having substituent(s), and
   R_{B} is a hydrogen atom, or a salt thereof,
[34] the compound of the above-mentioned [29] or [30], wherein R_{A} is a hydroxyl group optionally having substituent(s) selected from
   (1) an acyl group optionally having substituent(s); and
   (2) a sulfonyl group optionally having substituent(s), and
   R_{B} is a hydrogen atom, or a salt thereof,
[35] a compound represented by the formula:

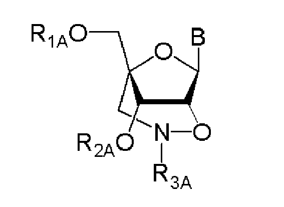

wherein
B is a purine ring group optionally having substituent(s);
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
(R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R_{2A} is a hydrogen atom or a 2-naphthylmethyl group; and
R_{3A} is a hydrogen atom, an alkyl group, an alkenyl group, a cycloalkyl group, an aryl group optionally having substituent(s), an aralkyl group optionally having substituent(s), an acyl group optionally having substituent(s), a sulfonyl group optionally having substituent(s), or a functional molecular unit substituent;
or a salt thereof,
[36] the compound of the above-mentioned [35], wherein R_{1A} is -Si(R_{8A})(R_{9A})(R_{10A})
   (R_{8A}, R_{9A} and R_{10A} are the same or different and each is
   (i) a lower alkyl group, or
   (ii) an aryl group optionally having substituent(s) selected from the group consisting of a lower alkyl group, a lower alkoxy group, a halogen atom and a cyano group),
   or a salt thereof,
[37] the compound of the above-mentioned [35] or [36], wherein R_{3A} is a hydrogen atom or an alkyl group having a carbon number of 1 - 5, or a salt thereof,
[38] the compound of any of the above-mentioned [29] to [37], wherein B is a 6-aminopurin-9-yl group wherein the amino group is optionally protected by a protecting group for nucleic acid synthesis, or a salt thereof,
[39] the compound of any of the above-mentioned [27] to [34], wherein R_{5A} is a p-toluenesulfonyl group, or a salt thereof.

### Effect of the Invention

The method of the present invention can produce an NC type nucleoside efficiently and conveniently in a high yield without an unnecessary protecting group-conversion step. In addition, the method of the present invention enables invertion of the steric chemistry of the 2'-position of an NC type nucleoside wherein B in the formula (I) is a purine group, whereby a purine NC type nucleoside can be produced efficiently in a high yield. Furthermore, using the intermediate compound of the present invention for the method, a purine NC type nucleoside can be produced efficiently and conveniently in a high yield.

### (Detailed Description of the Invention)

The definitions of the symbols and terms used in the present invention are explained in detail in the following.

Examples of the "halogen atom" include a fluorine atom, a chlorine atom, a bromine atom and an iodine atom. Preferred are a fluorine atom and a chlorine atom.

The "halogeno" or "halogenated" means that a substituent is substituted by the above-mentioned "halogen atom".

The "carboxylation" means that a substituent is substituted by a carboxy group.

The "nitration" means that a substituent is substituted by a nitro group.

The "lower" means, unless otherwise specified, that the carbon number is 1-6.

The "purine ring group" of the "purine ring group optionally having substituent(s)" includes any chemical structure capable of acting as or substituting a base having a purine ring generally known as a constituent component of nucleic acid (e.g., guanine, adenine), and other bases of nucleic acid components alanogous thereto. Preferable examples of the purine ring include adenine.
Examples of the "substituent" of the "purine ring group optionally having substituent(s)" include groups recited in the following group α. The purine ring group may have one or more (preferably 1 to 3) of such substituents at substitutable position(s).
Preferable examples of the "purine ring group optionally having substituent(s)" include a purine ring group optionally having substituent(s), which is bonded at the 9-position of the purine ring, more preferably, a purin-9-yl group optionally having substituent(s) selected from the following group α.

Group α: a hydroxyl group,
a hydroxyl group protected by a protecting group for nucleic acid synthesis,
an alkoxy group having a carbon number of 1 - 5,
a mercapto group,
a mercapto group protected by a protecting group for nucleic acid synthesis, an alkylthio group having a carbon number of 1 - 5,
an amino group,
an amino group protected by a protecting group for nucleic acid synthesis,
an amino group substituted by an alkyl group having a carbon number of 1 - 5, an alkyl group having a carbon number of 1 - 5, and
a halogen atom.

More preferable examples of the "purine ring group optionally having substituent(s)" include
a 6-aminopurin-9-yl group (i.e., adeninyl),
a 6-aminopurin-9-yl group wherein the amino group is protected by a protecting group for nucleic acid synthesis,
a 2,6-diaminopurin-9-yl group,
a 2-amino-6-chloropurin-9-yl group,
a 2-amino-6-chloropurin-9-yl group wherein the amino group is protected by a protecting group for nucleic acid synthesis,
a 2-amino-6-fluoropurin-9-yl group,
a 2-amino-6-fluoropurin-9-yl group wherein the amino group is protected by a protecting group for nucleic acid synthesis,
a 2-amino-6-bromopurin-9-yl group,
a 2-amino-6-bromopurin-9-yl group wherein the amino group is protected by a protecting group for nucleic acid synthesis,
a 2-amino-6-hydroxypurin-9-yl group (i.e., guaninyl),
a 2-amino-6-hydroxypurin-9-yl group wherein the amino group is protected by a protecting group for nucleic acid synthesis,
a 6-amino-2-methoxypurin-9-yl group,
a 6-amino-2-chloropurin-9-yl group,
a 6-amino-2-fluoropurin-9-yl group,
a 2,6-dimethoxypurin-9-yl group,
a 2,6-dichloropurin-9-yl group, and
a 6-mercaptopurine-9-yl group.
More preferable examples include
a 6-aminopurin-9-yl group (i.e., adeninyl),
a 6-benzoylaminopurin-9-yl group,
a 2-amino-6-hydroxypurin-9-yl group (i.e., guaninyl), and
a 2-isobutyrylamino-6-hydroxypurin-9-yl group.
Particularly preferable examples include a 6-aminopurin-9-yl group (i.e., adeninyl).

The "protecting group" of the "hydroxyl-protecting group for nucleic acid synthesis" and "hydroxyl group protected by a protecting group for nucleic acid synthesis" is not particularly limited as long as it can stably protect a hydroxyl group in nucleic acid synthesis. Specifically, it means a protecting group which is stable in acidic or neutral conditions, and can be cleaved by a chemical method such as hydrogenolysis, hydrolysis, electrolysis and photolysis.
Specific examples of the "protecting group" of the "hydroxyl-protecting group for nucleic acid synthesis" and "hydroxyl group protected by a protecting group for nucleic acid synthesis" include
(1) an aliphatic acyl group optionally having substituent(s)
   [for example, an alkyl-carbonyl group (e.g., formyl, acetyl, propionyl, butyryl, isobutyryl, pentanoyl, pivaloyl, valeryl, isovaleryl, octanoyl, nonanoyl, decanoyl, 3-methyl-nonanoyl, 8-methyl-nonanoyl, 3-ethyl-octanoyl, 3,7-dimethyl-octanoyl, undecanoyl, dodecanoyl, tridecanoyl, tetradecanoyl, pentadecanoyl, hexadecanoyl, 1-methyl-pentadecanoyl, 14-methyl-pentadecanoyl, 13,13-dimethyltetradecanoyl, heptadecanoyl, 15-methyl-hexadecanoyl, octadecanoyl, 1-methylheptadecanoyl, nonadecanoyl, eicosanoyl and heneicosanoyl),
   a carboxylated alkyl-carbonyl group (e.g., succinoyl, glutaroyl, adipoyl),
   a halogeno lower alkyl-carbonyl group (e.g., chloroacetyl, dichloroacetyl, trichloroacetyl, trifluoroacetyl),
   a lower alkoxy lower alkyl-carbonyl group (e.g., methoxyacetyl),
   an unsaturated alkyl-carbonyl group (e.g., lower alkenyl-carbonyl group, preferably, (E)-2-methyl-2-butenoyl)];
(2) a lower alkyl group
   [for example, methyl, ethyl, n-propyl, isopropyl, n-butyl, isobutyl, sec-butyl, tert-butyl, n-pentyl, isopentyl, 2-methyl-butyl, neopentyl, 1-ethyl-propyl, n-hexyl, isohexyl, 4-methyl-pentyl, 3-methyl-pentyl, 2-methyl-pentyl, 1-methyl-pentyl, 3,3-dimethyl-butyl, 2,2-dimethyl-butyl, 1,1-dimethyl-butyl, 1,2-dimethyl-butyl, 1,3-dimethyl-butyl, 2,3-dimethyl-butyl, 2-ethyl-butyl];
(3) a lower alkenyl group
   [for example, ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl, 5-hexenyl];
(4) an aromatic acyl group optionally having substituent(s)
   [for example, an aryl-carbonyl group (e.g., benzoyl, α-naphthoyl, β-naphthoyl),
   a halogenoaryl-carbonyl group (e.g., 2-bromobenzoyl, 4-chlorobenzoyl),
   a lower alkylated aryl-carbonyl group (e.g., 2,4,6-trimethyl-benzoyl, 4-toluoyl),
   a lower alkoxylated aryl-carbonyl group (e.g., p-methoxybenzoyl),
   a carboxylated aryl-carbonyl group (e.g., 2-carboxybenzoyl, 3-carboxybenzoyl, 4-carboxybenzoyl),
   a nitrated aryl-carbonyl group (e.g., 4-nitrobenzoyl, 2-nitrobenzoyl),
   a lower alkoxycarbonylated aryl-carbonyl group (e.g., 2-(methoxycarbonyl)benzoyl),
   an arylated aryl-carbonyl group (e.g., 4-phenylbenzoyl)];
(5) a tetrahydropyranyl group or a tetrahydrothiopyranyl group, each optionally substituted by 1 to 3 substituents selected from a halogen atom and a lower alkoxy group
   [for example, tetrahydropyran-2-yl, 3-bromotetrahydropyran-2-yl, 4-methoxytetrahydropyran-4-yl, tetrahydrothiopyran-4-yl, 4-methoxytetrahydrothiopyran-4-yl];
(6) a tetrahydrofuranyl group or a tetrahydrothiofuranyl group
   [for example, tetrahydrofuran-2-yl, tetrahydrothiofuran-2-yl];
(7) a lower alkoxymethyl group
   [for example, methoxymethyl, 1,1-dimethyl-1-methoxymethyl, ethoxymethyl, propoxymethyl, isopropoxymethyl, butoxymethyl, tert-butoxymethyl];
(8) a lower alkoxylated lower alkoxymethyl group
   [for example, 2-methoxyethoxymethyl];
(9) a halogeno lower alkoxymethyl group
   [for example, 2,2,2-trichloroethoxymethyl, bis(2-chloroethoxy)methyl];
(10) a lower alkoxylated ethyl group
   [for example, 1-ethoxyethyl, 1-(isopropoxy)ethyl];
(11) a halogenated ethyl group
   [for example, 2,2,2-trichloroethyl];
(12) a methyl group substituted by 1 to 3 aryl groups
   [for example, benzyl, α-naphthylmethyl, β-naphthylmethyl, diphenylmethyl, triphenylmethyl (i.e., trityl), α-naphthyldiphenylmethyl, 9-anthrylmethyl];
(13) a methyl group substituted by 1 to 3 aryl groups optionally having substituent(s) (at least one of said aryl groups has a substituent) (preferably, a methyl group substituted by 1 to 3 aryl groups optionally substituted by 1 to 3 substituents selected from the group consisting of a lower alkyl group, a lower alkoxy group, a halogen atom, a nitro group and a cyano group (at least one of said aryl groups has a substituent))
   [for example, 4-methylbenzyl, 2,4,6-trimethylbenzyl, 3,4,5-trimethylbenzyl, 4-methoxybenzyl (i.e., p-methoxybenzyl), monomethoxytrityl (e.g., 4-methoxytriphenylmethyl), dimethoxytrityl (e.g., 4,4'-dimethoxytriphenylmethyl), 2-nitrobenzyl, 4-nitrobenzyl, 4-chlorobenzyl, 4-bromobenzyl, 4-cyanobenzyl];
(14) a lower alkoxy-carbonyl group
   [for example, methoxycarbonyl, ethoxycarbonyl, tert-butoxycarbonyl, isobutoxycarbonyl];
(15) an aryl group substituted by 1 to 3 substituents selected from a halogen atom, a lower alkoxy group and a nitro group
   [for example, 4-chlorophenyl, 2-fluorophenyl, 4-methoxyphenyl, 4-nitrophenyl, 2,4-dinitrophenyl];
(16) a lower alkoxy-carbonyl group substituted by 1 to 3 substituents selected from a halogen atom and a tri-lower alkyl-silyl group
   [for example, 2,2,2-trichloroethoxycarbonyl, 2-trimethylsilylethoxycarbonyl];
(17) an alkenyloxy-carbonyl group
   [for example, vinyloxycarbonyl, allyloxycarbonyl]; and
(18) an aralkyloxy-carbonyl group wherein the aryl ring may be substituted by 1 to 3 substituents selected from lower alkoxy and a nitro group [for example, benzyloxycarbonyl, 4-methoxybenzyloxycarbonyl, 3,4-dimethoxybenzyloxycarbonyl, 2-nitrobenzyloxycarbonyl, 4-nitrobenzyloxycarbonyl];
   and the like.

Preferable examples of the "hydroxyl-protecting group for nucleic acid synthesis" include an aliphatic acyl group optionally having substituent(s),
an aromatic acyl group optionally having substituent(s),
a methyl group substituted by 1 to 3 aryl groups,
a methyl group substituted by 1 to 3 aryl groups optionally having substituent(s) (at least one of said aryl groups has a substituent) (preferably, a methyl group substituted by 1 to 3 aryl groups substituted by 1 to 3 substituents selected from the group consisting of a lower alkyl group, a lower alkoxy group, a halogen atom and a cyano group (at least one of said aryl groups has a substituent)) and the like.
More preferably, an acetyl group, a benzoyl group, a benzyl group, a p-methoxybenzoyl group, a p-methoxybenzyl group, a trityl group, a dimethoxytrityl group and a monomethoxytrityl group can be mentioned.
Preferable examples of the "protecting group" of the "hydroxyl group protected by a protecting group for nucleic acid synthesis" include an aliphatic acyl group optionally having substituent(s); an aromatic acyl group optionally having substituent(s); a methyl group substituted by 1 to 3 aryl groups; an aryl group substituted by 1 to 3 substituents selected from a halogen atom, a lower alkoxy group and a nitro group; a lower alkyl group; or a lower alkenyl group, more preferably, a benzoyl group, a benzyl group, a 2-chlorophenyl group, a 4-chlorophenyl group or a 2-propenyl group.

The "protecting group" of the "phosphate group protected by a protecting group for nucleic acid synthesis" is not particularly limited as long as it can stably protect a phosphate group in nucleic acid synthesis. Specifically, it means a protecting group which is stable in acidic or neutral conditions, and can be cleaved by a chemical method such as hydrogenolysis, hydrolysis, electrolysis and photolysis.
Specific examples of the "protecting group" include
(1) a lower alkyl group [for example, methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl];
(2) a cyanated lower alkyl group [for example, 2-cyanoethyl, 2-cyano-1,1-dimethylethyl];
(3) an ethyl group substituted by a substituted silyl group (e.g., a mono-lower alkyl-diarylsilyl group, a tri-lower alkyl silyl group, a triarylsilyl group) [for example, 2-methyldiphenylsilylethyl, 2-trimethylsilylethyl, 2-triphenylsilylethyl];
(4) a halogenated lower alkyl group [for example, 2,2,2-trichloroethyl, 2,2,2-tribromoethyl, 2,2,2-trifluoroethyl, 2,2,2-trichloro-1,1-dimethylethyl];
(5) a lower alkenyl group [for example, ethenyl, 1-propenyl, 2-propenyl, 1-methyl-2-propenyl, 1-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl, 5-hexenyl];
(6) a cycloalkyl group [for example, cyclopropyl, cyclobutyl, cyclopentyl, cyclohexyl, cycloheptyl, norbornyl, adamantyl];
(7) a cyanated lower alkenyl group [for example, 2-cyanobutenyl];
(8) an aralkyl group [for example, benzyl, α-naphthylmethyl, β-naphthylmethyl, indenylmethyl, phenanthrenylmethyl, anthracenylmethyl, diphenylmethyl, triphenylmethyl, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl, 6-naphthylhexyl];
(9) an aralkyl group wherein the aryl ring is substituted by substituents (e.g., 1 to 3) selected from the group consisting of a nitro group and a halogen atom [for example, 4-chlorobenzyl, 2-(4-nitrophenyl)ethyl, o-nitrobenzyl, 4-nitrobenzyl, 2,4-dinitrobenzyl, 4-chloro-2-nitrobenzyl];
(10) an aryl group [for example, phenyl, indenyl, naphthyl, phenanthrenyl, anthracenyl]; and
(11) an aryl group substituted by substituents (e.g., 1 to 3) selected from the group consisting of a lower alkyl group, a halogen atom and a nitro group [for example, 2-methylphenyl, 2,6-dimethylphenyl, 2-chlorophenyl, 4-chlorophenyl, 2,4-dichlorophenyl, 2,5-dichlorophenyl, 2-bromophenyl, 4-nitrophenyl, 4-chloro-2-nitrophenyl].
Preferable examples of the "protecting group" of the "phosphate group protected by a protecting group for nucleic acid synthesis" include a lower alkyl group,
a cyanated lower alkyl group,
an aralkyl group,
an aralkyl group wherein the aryl ring is substituted by 1 to 3 substituents selected from the group consisting of a nitro group and a halogen atom, and an aryl group substituted by substituents selected from the group consisting of a lower alkyl group, a halogen atom and a nitro group.
More preferable examples include a 2-cyanoethyl group, a 2,2,2-trichloroethyl group, a benzyl group, a 2-chlorophenyl group and a 4-chlorophenyl group.

The "protecting group" of the "mercapto group protected by a protecting group for nucleic acid synthesis" is not particularly limited as long as it can stably protect a mercapto group in nucleic acid synthesis. Specifically, it means a protecting group which is stable in acidic or neutral conditions, and can be cleaved by a chemical method such as hydrogenolysis, hydrolysis, electrolysis and photolysis.
Specific examples of the "protecting group" include those exemplified as the protecting group of the above-mentioned "hydroxyl group protected by a protecting group for nucleic acid synthesis", as well as a group forming a disulfide group [for example, an alkylthio group (e.g., methylthio, ethylthio, tert-butylthio), an aralkylthio group (e.g., benzylthio) etc.]. Of these, preferred are an aliphatic acyl group, an aromatic acyl group, and a methyl group substituted by 1 to 3 aryl groups, and more preferred are a benzoyl group and a benzyl group.

The "protecting group" of the "amino group protected by a protecting
group for nucleic acid synthesis" is not particularly limited as long as it can stably protect an amino group in nucleic acid synthesis. Specifically, it means a protecting group which is stable in acidic or neutral conditions, and can be cleaved by a chemical method such as hydrogenolysis, hydrolysis, electrolysis and photolysis. The "amino group protected by a protecting group for nucleic acid synthesis" means that the amino group in the structure is protected by the protecting group.
Specific examples of the "protecting group" include
(1) an aliphatic acyl group optionally having substituent(s)
   [for example, an alkyl-carbonyl group (e.g., formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl, heneicosanoyl),
   carboxylated alkyl-carbonyl group (e.g., succinoyl, glutaroyl, adipoyl),
   halogeno lower alkyl-carbonyl group (e.g., chloroacetyl, dichloroacetyl, trichloroacetyl, trifluoroacetyl),
   lower alkoxy lower alkyl-carbonyl group (e.g., methoxyacetyl),
   unsaturated alkyl-carbonyl group (e.g., lower alkenyl-carbonyl group, preferably,
   (E)-2-methyl-2-butenoyl)];
(2) an aromatic acyl group optionally having substituent(s)
   [for example, an aryl-carbonyl group (e.g., benzoyl, α-naphthoyl, β-naphthoyl),
   a halogenoaryl-carbonyl group (e.g., 2-bromobenzoyl, 4-chlorobenzoyl),
   a lower alkylated aryl-carbonyl group (e.g., 2,4,6-trimethylbenzoyl, 4-toluoyl),
   a lower alkoxylated aryl-carbonyl group (e.g., 4-anisoyl),
   a carboxylated aryl-carbonyl group (e.g., 2-carboxybenzoyl, 3-carboxybenzoyl, 4-carboxybenzoyl),
   a nitrated aryl-carbonyl group (e.g., 4-nitrobenzoyl, 2-nitrobenzoyl),
   a lower alkoxycarbonylated aryl-carbonyl group (e.g., 2-(methoxycarbonyl)benzoyl),
   an arylated aryl-carbonyl group (e.g., 4-phenylbenzoyl)etc.];
(3) a lower alkoxy-carbonyl group
   [for example, methoxycarbonyl, ethoxycarbonyl, tert-butoxycarbonyl, isobutoxycarbonyl];
(4) a lower alkoxy-carbonyl group substituted by 1 to 3 substituents selected from a halogen atom and a tri-lower alkylsilyl group [for example, 2,2,2-trichloroethoxycarbonyl, 2-trimethylsilylethoxycarbonyl];
(5) an alkenyloxy-carbonyl group [for example, vinyloxycarbonyl, allyloxycarbonyl]; and
(6) an aralkyloxy-carbonyl group wherein the aryl ring is optionally substituted by (e.g., 1 or 2) substituents selected from the group consisting of a lower alkoxy and a nitro group [for example, benzyloxycarbonyl, 4-methoxybenzyloxycarbonyl, 2-nitrobenzyloxycarbonyl, 4-nitrobenzyloxycarbonyl].
Particularly preferred are an aliphatic acyl group and an aromatic acyl group, and more preferred is a benzoyl group.

The "alkyl- group" is a straight chain or branched chain alkyl group having a carbon number of 1 - 20, namely, a straight chain or branched chain alkyl group having a carbon number of 1 - 6 or a straight chain or branched chain alkyl group having a carbon number of 7 - 20. Particularly preferred is a straight chain or branched chain alkyl group having a carbon number of 1 - 6.
Examples of the straight chain or branched chain alkyl group having a carbon number of 1 - 6 include methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl and the like.
Examples of the straight chain or branched chain alkyl group having a carbon number of 7 - 20 include heptyl, octyl, nonyl, decyl and the like.

The "lower alkyl group" is a straight chain or branched chain alkyl group having a carbon number of 1 - 6. Specific examples of the "lower alkyl group" include those similar to the groups exemplified as the above-mentioned "straight chain or branched chain alkyl group having a carbon number of 1 - 6".

The "lower alkylation" means that the substituent is substituted by the above-mentioned "lower alkyl group".

Specific examples of the "alkyl- group having a carbon number of 1 - 5" include methyl, ethyl, propyl, isopropyl, isopropyl, butyl, isobutyl, sec-butyl, tert-butyl, n-pentyl and the like, with preference given to methyl and ethyl.

The alkyl-carbonyl group is a carbonyl group substituted by the above-mentioned "alkyl- group".

The "alkenyl group" is a straight chain or branched chain alkenyl group having a carbon number of 2 - 20. Preferred is a straight chain or branched chain alkenyl group having a carbon number of 2 - 6 (which is also referred to as a lower alkenyl group in the present specification), geranyl, farnesyl and the like, and more preferred is a straight chain or branched chain alkenyl group having a carbon number of 2 to 6.
Specific examples of the "straight chain or branched chain alkenyl group having a carbon number of 2 - 6" include ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl, 5-hexenyl and the like.

Examples of the "alkenyl group having a carbon number of 2 - 5" include those exemplified as the above-mentioned "alkenyl group", which have a carbon number of 2-5.

The "alkoxy group" is a straight chain or branched chain alkoxy group having a carbon number of 1 - 10. Specific examples thereof include methoxy, ethoxy, n-propoxy, isopropoxy, n-butoxy, isobutoxy, sec-butoxy, tert-butoxy, n-pentoxy, hexoxy, heptoxy, octoxy, nonoxy, decyloxy and the like.
Examples of the "lower alkoxy group" include an alkoxy group having a carbon number of 1 - 5. Examples of the "alkoxy group having a carbon number of 1 - 5" include methoxy, ethoxy, n-propoxy, isopropoxy, n-butoxy, isobutoxy, sec-butoxy, tert-butoxy and n-pentoxy, with preference given to a methoxy group and an ethoxy group.

The "lower alkoxylation" means that the substituent is substituted by the above-mentioned "lower alkoxy group".

The "lower alkoxy-carbonyl group" is carbonyl substituted by the above-mentioned "lower alkoxy group", which specifically includes methoxycarbonyl, ethoxycarbonyl and the like.

The "lower alkoxy-carbonylation" means that the substituent is substituted by the above-mentioned "lower alkoxy-carbonyl group".

The "aryloxy group" is an oxy group substituted by the following "arty! group", which specifically includes a phenyloxy group and the like.

The "aryl-carbonyl (group)" is a carbonyl group substituted by the following "arty! group".

The "arylthio (group)" is a mercapto group substituted by the following "arty! group".

The "arylation" means that the substituent is substituted by the following "aryl group".

Preferable examples of the "cycloalkyl group" include a cycloalkyl group having a carbon number of 3 - 10. More preferred is, for example, a cycloalkyl group having a carbon number of 3 - 8.
Preferable examples of the "cycloalkyl group" include cyclopropyl, cyclobutyl, cyclopentyl, cyclohexyl, cycloheptyl, cyclooctyl, norbornyl, adamantyl and the like. More preferred are, for example, cyclopropyl, cyclobutyl, cyclopentyl, cyclohexyl, cycloheptyl, cyclooctyl and the like.

The "nonaromatic heterocyclic group" is one wherein one or more methylene on the ring of a cycloalkyl group is(are) substituted by an oxygen atom, a sulfur atom, or a nitrogen atom substituted by an alkyl group.
Preferable examples of the "nonaromatic heterocyclic group" include a tetrahydropyranyl group and the like.

The "aryl group" is a monovalent group having a carbon number of 6 - 14 obtained by removing one hydrogen atom from the aromatic hydrocarbon ring (e.g., benzene, naphthalene, phenanthrene, anthracene, inden). Examples of thereof include a phenyl group, an indenyl group, a naphthyl group, a phenanthrenyl group, an anthracenyl group (i.e., anthryl group) and the like.
Examples of the "substituents" of the "aryl group optionally having substituent(s)" include a lower alkyl group, an alkoxy group, a halogen atom, a cyano group, a hydroxyl group, an aryloxy group, an amino group, a nitro group, a trifluoromethyl group, a phenyl group and the like. Preferred are a lower alkyl group (e.g., methyl), a lower alkoxy group (e.g., methoxy), a halogen atom (e.g., chlorine atom, bromine atom) and a cyano group. When two or more substituents are present, they may be of the same kind or different kinds.
Examples of the "aryl group optionally having substituent(s)" include phenyl, indenyl, naphthyl, phenanthrenyl, anthracenyl, 2-methylphenyl, 4-methylphenyl, 2,6-dimethylphenyl, 2-chlorophenyl, 4-chlorophenyl, 2,4-dichlorophenyl, 2,5-dichlorophenyl, 2-bromophenyl, 2-methoxyphenyl, 3-methoxyphenyl, 4-methoxyphenyl, 4-chloro-2-nitrophenyl, 4-nitrophenyl, 2,4-dinitrophenyl, biphenyl and the like. Preferred are a phenyl group substituted by a halogen atom, a lower alkoxy group, or a nitro group, a phenyl group and the like.

Examples of the "substituent" of the "aryl group having a carbon number of 6 - 14 and optionally having substituent(s)" include those similar to the substituent exemplified for the above-mentioned "aryl group optionally having substituent(s)".
Examples of the "aryl group having a carbon number of 6 - 14 and optionally having substituent(s)" include those similar to the above-mentioned "aryl group optionally having (1 to 3 ) substituent(s)".

Examples of the "aralkyl group" of the "aralkyl group optionally having substituent(s)" include an alkyl group having a carbon number of 1 - 6 substituted by the above-mentioned "aryl group" and the like. Preferred are an alkyl group having a carbon number of 1 - 6, which is substituted by 1 to 3 aryl groups, and the like, and more preferred are a methyl group substituted by 1 to 3 aryl groups and the like.
Examples of the "substituent" of the "aralkyl group optionally having substituent(s)" include a lower alkyl group, a lower alkoxy group, a halogen atom, a cyano group and the like.
Preferable examples of the "aralkyl group optionally having substituent(s)" include
(1) a methyl group substituted by 1 to 3 aryl groups optionally having substituent(s), more preferably a methyl group substituted by 1 to 3 aryl groups optionally having substituent(s) [for example, a methyl group substituted by 1 to 3 aryl groups (e.g., benzyl, 1-naphthylmethyl, 2-naphthylmethyl, indenylmethyl, phenanthrenylmethyl, anthracenylmethyl, diphenylmethyl, triphenylmethyl (i.e., trityl), 1-naphthyldiphenylmethyl, 9-anthrylmethyl);
   a methyl group substituted by 1 to 3 aryl groups having 1 to 3 substituents selected from the group consisting of a lower alkyl group, a lower alkoxy group, a halogen atom and a cyano group (e.g., 4-methylbenzyl, 2,4,6-trimethylbenzyl, 3,4,5-trimethylbenzyl, 4-methoxybenzyl(p-methoxybenzyl), monomethoxytrityl (e.g., 4-methoxytriphenylmethyl), dimethoxytrityl (e.g., 4,4'-dimethoxytriphenylmethyl), 2-nitrobenzyl, 4-nitrobenzyl, 4-chlorobenzyl, 4-bromobenzyl, 4-cyanobenzyl)]; and
(2) an alkyl group having a carbon number of 2 - 6, which is substituted by an aryl group
   [for example, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylhexyl, 2-naphthylhexyl, 3-naphthylhexyl, 4-naphthylhexyl, 5-naphthylhexyl, 6-naphthylhexyl];
   and the like.
Particularly preferred are a methyl group substituted by 1 to 3 aryl groups and a methyl group substituted by 1 to 3 aryl groups substituted by at least one substituent selected from the group consisting of a lower alkyl group, a lower alkoxy group, a halogen atom and a cyano group, more preferred are 4-methoxytriphenylmethyl and 4,4'-dimethoxytriphenylmethyl.

Examples of the "acryl group optionally having substituent(s)" include the following aliphatic acyl group optionally having substituent(s), and an aromatic acyl group optionally having substituent(s).
(1) an aliphatic acyl group optionally having substituent(s)
   [for example, an alkyl-carbonyl group (e.g., formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl and heneicosanoyl),
   a carboxylated alkyl-carbonyl group (e.g., succinoyl, glutaroyl, adipoyl),
   a halogeno lower alkyl-carbonyl group (e.g., chloroacetyl, dichloroacetyl, trichloroacetyl, trifluoroacetyl),
   a lower alkoxy lower alkyl-carbonyl group (e.g., methoxyacetyl),
   an aryloxy lower alkyl-carbonyl group (e.g., phenoxyacetyl),
   an unsaturated alkyl-carbonyl group (e.g., lower alkenyl-carbonyl group, preferably, (E)-2-methyl-2-butenoylthenoyl)];
(2) an aromatic acyl group optionally having substituent(s)
   [for example, an aryl-carbonyl group (e.g., benzoyl, α-naphthoyl, β-naphthoyl), a halogenoaryl-carbonyl group (e.g., 2-bromobenzoyl, 4-chlorobenzoyl),
   a lower alkylated aryl-carbonyl group (e.g., 2,4,6-trimethylbenzoyl, 4-toluoyl),
   a lower alkoxylated aryl-carbonyl group (e.g., 4-anisoyl),
   a carboxylated aryl-carbonyl group (e.g., 2-carboxybenzoyl, 3-carboxybenzoyl, 4-carboxybenzoyl), a nitrated aryl-carbonyl group (e.g., 4-nitrobenzoyl, 2-nitrobenzoyl),
   a lower alkoxycarbonylated aryl-carbonyl group (e.g., 2-(methoxycarbonyl)benzoyl),
   an arylated aryl-carbonyl group (e.g., 4-phenylbenzoyl)];
   and the like.
Examples of the "substituents" of the "acryl group optionally having substituent(s)" include
(1) a carboxy group,
(2) a halogen atom,
(3) a lower alkoxy group,
(4) an aryloxy group,
(5) a lower alkyl group,
(6) a nitro group,
(7) a lower alkoxy-carbonyl group,
(8) an aryl group and the like.
Preferable examples of the "acryl group optionally having substituent(s)" include formyl, acetyl, propionyl, butyryl, isobutyryl, pentanoyl, pivaloyl, benzoyl, phenoxyacetyl group and the like.

Examples of the "aliphatic acyl group optionally having substituent(s)" include those similar to the examples of the "aliphatic acyl group optionally having substituent(s)" of the above-mentioned "acryl group optionally having substituent(s)".
Examples of the "substituents" of the "aliphatic acyl group optionally having substituent(s)" include those similar to the examples of the substituent of the above-mentioned "acryl group optionally having substituent(s)".

Examples of the "aliphatic acyl group having a carbon number of 1 - 5 and optionally having substituent(s)" include those wherein the aliphatic acyl group has a carbon number of 1 - 5 from among those exemplified as the "aliphatic acyl group optionally having substituent(s)" of the above-mentioned "acryl group optionally having substituent(s)".
Examples of the "substituents" of the "aliphatic acyl group having a carbon number of 1 - 5 and optionally having substituent(s)" include those similar to the examples of the substituent of the above-mentioned "acryl group optionally having substituent(s)".

Examples of the "alkyl- having a carbon number of 1 - 6 - carbonyl group optionally having substituent(s)" include those wherein the acyl group is an alkyl having a carbon number of 1 - 6 -carbonyl group from among those exemplified as the above-mentioned "acryl group optionally having substituent(s)".
Examples of the "substituents" of the "alkyl- group having a carbon number of 1 - 6 - carbonyl group optionally having substituent(s)" include those similar to the examples of the substituent of the above-mentioned "acryl group optionally having substituent(s)".
The "alkyl- having a carbon number of 1 - 6 - carbonyl group optionally having substituent(s)" is preferably an acetyl group.

Examples of the "aromatic acyl group optionally having substituent(s)" include those similar to the examples of the "aromatic acyl group optionally having substituent(s)" of the above-mentioned "acryl group optionally having substituent(s)".
Examples of the "substituents" of the "aromatic acyl group optionally having substituent(s)" include those similar to the examples of the substituent of the above-mentioned "acryl group optionally having substituent(s)".

Examples of the "substituent" of the "sulfonyl group optionally having substituent(s)" include
an aliphatic group optionally having substituent(s) (e.g., substituent selected from the above-mentioned "group α" (excluding an alkyl group having a carbon number of 1 - 5), preferably a halogen atom, more preferably a fluorine atom)
[for example, a straight chain or branched alkyl group having a carbon number of 1 - 6 (e.g., methyl, ethyl)], and
an aromatic group optionally having substituent(s) (e.g., substituent selected from the above-mentioned "group α", preferably an alkyl group having a carbon number of 1 - 5, more preferably methyl) [for example, an aryl group (e.g., phenyl)].
Examples of the "sulfonyl group optionally having substituent(s)" include (1) an aliphatic sulfonyl group optionally having substituent(s) [for example, a lower aliphatic sulfonyl group optionally having substituent(s) (e.g., methanesulfonyl, ethanesulfonyl, trifluoromethanesulfonyl)]; and (2) an aromatic (e.g., aryl group (e.g., phenyl)) sulfonyl group optionally having a substituent (e.g., substituent selected from the above-mentioned "group α", preferably an alkyl group having a carbon number of 1 - 5, more preferably methyl) [for example, a sulfonyl group substituted by an aryl group optionally having substituent(s) (e.g., phenylsulfonyl, p-toluenesulfonyl)].
Here, the "lower aliphatic sulfonyl group optionally having substituent(s)" means a sulfonyl group optionally having a straight chain or branched alkyl group having a carbon number of 1 - 6 (e.g., methyl, ethyl) substituted by a substituent (e.g., substituent selected from the above-mentioned "group α" (excluding alkyl group having a carbon number of 1 - 5), preferably a halogen atom, more preferably a fluorine atom).
Particularly preferred are methanesulfonyl, trifluoromethanesulfonyl, phenylsulfonyl and p-toluenesulfonyl.

Examples of the "aliphatic sulfonyl group optionally having substituent(s)" include the above-mentioned "lower aliphatic sulfonyl group optionally having substituent(s)".

Examples of the "lower aliphatic sulfonyl group optionally having substituent(s)" include those similar to the groups exemplified as the "lower aliphatic sulfonyl group optionally having substituent(s)" of the above-mentioned "sulfonyl group optionally having substituent(s)".

Examples of the "alkylsulfonyl group" of the "alkylsulfonyl group optionally substituted by a halogen atom" include a sulfonyl group substituted by an alkyl group. Preferred is a sulfonyl group substituted by a lower alkyl group.

The "alkylsulfonyl group optionally substituted by a halogen atom" is preferably "a lower alkylsulfonyl group optionally substituted by a halogen atom", particularly preferably trifluoromethanesulfonyl.

Examples of the "aromatic sulfonyl group optionally having substituent(s)" include those similar to the examples of the "aromatic sulfonyl group optionally having substituent(s)" of the above-mentioned "sulfonyl group optionally having substituent(s)", preferably "a phenylsulfonyl group optionally having substituent(s)".

Examples of the "substituents" of the "aromatic sulfonyl group optionally having substituent(s)" and "phenylsulfonyl group optionally having substituent(s)" include those similar to the examples of the above-mentioned group α. Particularly preferred is an alkyl group having a carbon number of 1 - 5, more preferably, a methyl group.
Preferable examples of the "aromatic sulfonyl group optionally having substituent(s)" and "phenylsulfonyl group optionally having substituent(s)" include a p-toluenesulfonyl group.

Examples of the "substituents" of the "sibyl group optionally having substituent(s)" include a lower alkyl group, an aryl group optionally having substituent(s) and the like. Preferred are an aryl group (e.g., phenyl) optionally having substituent(s) selected from the group consisting of
(i) a lower alkyl group (e.g., methyl, ethyl, isopropyl, tert-butyl),
(ii) a lower alkyl group, a lower alkoxy group, a halogen atom and a cyano group
   and the like. The silyl group optionally has 1 - 3 substituents at substitutable position(s).
Preferable examples of the "sibyl group optionally having substituent(s)" include -Si(R₈)(R₉)(R₁₀)

wherein R₈, R₉ and R₁₀ are the same or different and each is
(i) a lower alkyl group, or
(ii) an aryl group optionally having substituent(s) selected from the group consisting of a lower alkyl group, a lower alkoxy group, a halogen atom and a cyano group.
Particularly preferred are trimethylsilyl, triethylsilyl, isopropyldimethylsilyl, tert-butyldimethylsilyl, methyldiisopropylsilyl, methyldi-tert-butylsilyl, triisopropylsilyl, diphenylmethylsilyl, diphenylbutylsilyl, butyldiphenylbutylsilyl, diphenylisopropylsilyl, phenyldiisopropylsilyl, tert-butyldiphenylsilyl and the like.
More preferred are trimethylsilyl, triethylsilyl, triisopropylsilyl, tert-butyldimethylsilyl, tert-butyldiphenylsilyl and the like, and particularly preferred are trimethylsilyl, tert-butyldiphenylsilyl and the like. Especially, tert-butyldiphenylsilyl is preferable.

Preferable examples of the "substituents" of the "hydroxyl group optionally having substituent(s)" include
(1) an acyl group optionally having substituent(s);
(2) a sulfonyl group optionally having substituent(s); and
(3) a group represented by the formula -NR_{11A}R_{12A}
   wherein R_{11A} and R_{12A} are the same or different and each is
   (i) a hydrogen atom,
   (ii) a hydrocarbon group optionally having substituent(s),
   (iii) a heterocyclic group optionally having substituent(s),
   (iv) an acyl group optionally having substituent(s), or
   (v) a sulfonyl group optionally having substituent(s), or
R_{11A} and R_{12A} form, together with the adjacent nitrogen atom, a cyclic group optionally having substituent(s).
Preferable examples of the "hydroxyl group optionally having substituent(s)" include a hydroxyl group, a trifluoromethanesulfonyloxy, acetyloxy and phthalimidoxy.

Examples of the "hydrocarbon group" of the "hydrocarbon group optionally having substituent(s)" for R_{11A} or R_{12A} include those similar to the groups exemplified as the aforementioned "alkyl- group", "alkenyl group", "cycloalkyl group" and "aryl group". Examples of the "substituent" of the "hydrocarbon group optionally having substituent(s)" include those similar to the groups exemplified as the above-mentioned "group α".

Examples of the "heterocyclic group" of the "heterocyclic group optionally having substituent(s)" for R_{11A} or R_{12A} include a monocyclic aromatic heterocyclic group, a fused aromatic heterocyclic group and a nonaromatic heterocyclic group.
Examples of the "monocyclic aromatic heterocyclic group" include a 5- to 7-membered monocyclic aromatic heterocyclic group containing, as a ring constituting atom besides carbon atom, 1 to 4 hetero atoms selected from an oxygen atom, a sulfur atom and a nitrogen atom, and the like. Examples of the "monocyclic aromatic heterocyclic group" include furyl, thienyl, pyridyl, pyrimidinyl, pyridazinyl, pyrazinyl, pyrrolyl, imidazolyl, pyrazolyl, thiazolyl, isothiazolyl, oxazolyl, isoxazolyl, oxadiazolyl, thiadiazolyl, triazolyl, tetrazolyl, triazinyl and the like.
The "fused aromatic heterocyclic group" is, for example, a 9- to 12-membered fused aromatic heterocyclic group wherein a 5- to 7-membered monocyclic aromatic heterocyclic group containing, as a ring constituting atom besides carbon atom, 1 to 4 hetero atoms selected from an oxygen atom, a sulfur atom and a nitrogen atom, etc. and C₆-₁₀ aryl and the like are fused; a 8- to 12-membered fused aromatic heterocyclic group wherein a 5- to 7-membered monocyclic aromatic heterocyclic groups are fused to each other, and the like. Examples of the "fused aromatic heterocyclic group" include quinolyl, isoquinolyl, quinazolyl, quinoxalyl, benzofuryl, isobenzofuryl, benzothienyl, isobenzothienyl, benzoxazolyl, benzothiazolyl, benzimidazolyl, indolyl, isoindolyl, indazolyl, pyrrolopyrazinyl, imidazopyridyl, imidazopyrazinyl, benzisoxazolyl, benzotriazolyl, benzopyrazolyl, pyrazolopyridyl, pyrazolothienyl, pyrazolotriazinyl and the like.
Examples of the "nonaromatic heterocyclic group" include those similar to the groups exemplified as the aforementioned "nonaromatic heterocyclic group".

Examples of the "cyclic group" of the cyclic group optionally having substituent(s) that R_{11A} and R_{12A} form together with the adjacent nitrogen atom include
a 5- to 7-membered monocyclic aromatic heterocyclic group containing, as a ring-constituting atom besides a carbon atom and one nitrogen atom, 1 to 3 hetero atoms selected from an oxygen atom, a sulfur atom and a nitrogen atom;
a 9- to 12-membered fused aromatic heterocyclic group wherein the monocyclic aromatic heterocyclic group etc. and C₆-₁₀ aryl and the like are fused; and a 8- to 12-membered fused aromatic heterocyclic group wherein the monocyclic aromatic heterocyclic groups are fused to each other;
and the like.
Examples of the monocyclic aromatic heterocyclic group include those exemplified as the "monocyclic aromatic heterocyclic group" of the above-mentioned "heterocyclic group", which contain, as a ring-constituting atom besides a carbon atom and one nitrogen atom, 1 to 3 hetero atoms selected from an oxygen atom, a sulfur atom and a nitrogen atom.
Examples of the "fused aromatic heterocyclic group" include those exemplified as the "fused aromatic heterocyclic group" of the above-mentioned "heterocyclic group" wherein (1) a 5- to 7-membered monocyclic aromatic heterocyclic group containing, as a ring-constituting atom besides a carbon atom and one nitrogen atom, 1 to 3 hetero atoms selected from an oxygen atom, a sulfur atom and a nitrogen atom, and C₆-₁₀ aryl and the like are fused, and (2) 5- to 7-membered monocyclic aromatic heterocyclic groups containing, as a ring-constituting atom besides a carbon atom and one nitrogen atom, 1 to 3 hetero atoms selected from an oxygen atom, a sulfur atom and a nitrogen atom are fused to each other. Particularly preferred is isoindolyl.
Examples of the "substituent" of the cyclic group optionally having substituent(s) that R_{11A} and R_{12A} form together with the adjacent nitrogen atom include the groups exemplified as the aforementioned "group α", and an oxo group. More preferred is an oxo group.
Examples of the "cyclic group optionally having substituent(s)" of the cyclic group optionally having substituent(s) formed by R_{11A} and R_{12A} together with the adjacent nitrogen atom preferably include phthalimido.

Examples of the "alkylthio group having a carbon number of 1 - 5" include methylthio, ethylthio, propylthio, isopropylthio, butylthio, isobutylthio, sec-butylthio, tert-butylthio, n-pentylthio and the like. Particularly preferred are methylthio, ethylthio and the like.

Examples of the "cyanoalkoxy group having a carbon number of 1 - 6" include the above-mentioned "alkoxy group having a carbon number of 1 - 5" substituted by a cyano group and the like.
Examples of the "cyanoalkoxy group having a carbon number of 1 - 6" preferably include cyanomethoxy, 2-cyanoethoxy, 3-cyanopropoxy, 4-cyanobutoxy, 3-cyano-2-methylpropoxy, 1-cyanomethyl-1,1-dimethylmethoxy and the like. More preferred is a 2-cyanoethoxy group.

Examples of the "amino group substituted by an alkyl group having a carbon number of 1 - 5" include methylamino, ethylamino, propylamino, isopropylamino, butylamino, isobutylamino, sec-butylamino, tert-butylamino, dimethylamino, diethylamino, dipropylamino, diisopropylamino, dibutylamino, diisobutylamino, di(sec-butyl)amino and di(tert-butyl)amino. Particularly preferred are methylamino, ethylamino, dimethylamino, diethylamino, diisopropylamino and the like.

The "functional molecular unit substituent" includes labeling molecules (e.g., fluorescence□luminescence-labeling molecule, chemical luminescence-labeling molecule, molecular kind including a radioisotope etc.), a functional group having nucleic acid cleavage activity (e.g., molecule having DNA or RNA cleavage activity), intracellular or nuclear localization signal peptide and the like.

Examples of the "solvent" include hydrocarbon solvents (e.g., benzene, toluene, xylene, hexane, heptane), halogenated hydrocarbon solvents (e.g., dichloromethane, chloroform), ether solvents (e.g., diethyl ether, diisopropyl ether, tert-butyl methyl ether, tetrahydrofuran, dioxane, dimethoxyethane), nitrile solvents (e.g., acetonitrile), amide solvents (e.g., N,N-dimethylformamide, N,N-dimethylacetamide), ester solvents (e.g., ethyl acetate), sulfoxide solvents (e.g., dimethyl sulfoxide), and the like. Two or more kinds of these solvents may be used as a mixture in an appropriate ratio.
As the solvent, preferred are sulfoxide solvents, and more preferred is dimethyl sulfoxide.

Examples of the "alkali metal" of the "organic acid salt of alkali metal" include lithium, sodium, potassium, rubidium, cesium and the like. Particularly preferred is cesium.
Preferable examples of the "organic acid salt" of the "organic acid salt of alkali metal" include acetate, trifluoroacetate, methanesulfonate, benzenesulfonate, p-toluenesulfonate and the like. Particularly preferred is acetate.
As the "organic acid salt of alkali metal", preferred is "alkali metal acetate" and more preferred is cesium acetate.

The "room temperature" is generally 0 - 35°C, preferably 20 - 25°C.

The above-mentioned compounds (I), (II) and (III), and the intermediate compounds described above (sometimes indicated as the compound of the present invention in the present specification) may be present as salts. Preferable examples of the salt include metal salts such as alkali metal salts (e.g., sodium salt, potassium salt and lithium salt), alkaline earth metal salts (e.g., calcium salt and magnesium salt), aluminum salt, iron salt, zinc salt, copper salt, nickel salt, cobalt salt and the like; amine salts such as inorganic salts (e.g., ammonium salt), organic salts (e.g., tert-octylamine salt, dibenzylamine salt, morpholine salt, glucosamine salt, phenylglycine alkylester salt, ethylenediamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzyl-phenethylamine salt, piperazine salt, tetramethylammonium salt and tris(hydroxymethyl)aminomethane salt) and the like; inorganic acid salts such as hydrohalides (e.g., hydrofluoride, hydrochloride, hydrobromide, hydroiodide), nitrate, perchlorate, sulfate, phosphate and the like; organic acid salts such as lower alkanesulfonates (e.g., methanesulfonate, trifluoromethanesulfonate, ethanesulfonate), arylsulfonates (e.g., benzenesulfonate, p-toluenesulfonate), acetate, maleate, fumarate, succinate, citrate, tartrate, oxalate, maleate and the like; and salts with amino acid such as glycinate, lysinate, argininate, ornithinate, glutamate and aspartate.

When the compound of the present invention is obtained in a free form, it can be converted to a desired salt by a method known *per se* or a method analogous thereto. Conversely, when the compound is obtained as a salt, it can be converted to a free form or a desired other salt by a method known *per* se or a method analogous thereto.

When the compound of the present invention or a salt thereof has isomers such as optical isomer, stereoisomer, positional isomer, rotational isomer and the like, any isomers and mixtures are encompassed in the compound of the present invention or a salt thereof. These isomers can be obtained as independent products by a synthesis means or a separation means (concentration, solvent extraction, column chromatography, recrystallization and the like) known *per se.*

The compound of the present invention or a salt thereof may be a crystal or a solvate, both of which are encompassed in the compound of the present invention or a salt thereof.
A compound labeled with an isotope (e.g., ²H ³H, ¹⁴C, ³⁵S, ¹²⁵I etc.) is also encompassed in the compound of the present invention or a salt thereof.

When the method of the present invention is practiced, the compound of the present invention and a salt thereof are preferable examples. Of these, the following compounds (1) - (8), (A-1), (A-2) and (A-3), and salts thereof are more preferable.

### [Compound (1)]

Compounds (I), (II) and (III), wherein
R₁ is
an aliphatic acyl group optionally having substituent(s),
an aromatic acyl group optionally having substituent(s),
an aliphatic sulfonyl group optionally having substituent(s),
an aromatic sulfonyl group optionally having substituent(s),
a methyl group substituted by 1 to 3 aryl groups optionally having 1 to 3 substituents (e.g., a methyl group substituted by 1 to 3 aryl groups optionally having 1 to 3 substituents selected from the group consisting of a lower alkyl group, a lower alkoxy group, a halogen atom and a cyano group), or
-Si(R₈)(R₉)(R₁₀)
wherein R₈, R₉ and R₁₀ are the same or different and each is
(i) a lower alkyl group, or
(ii) an aryl group optionally having substituent(s) selected from the group consisting of a lower alkyl group, a lower alkoxy group, a halogen atom and a cyano group,
or a salt thereof.

### [Compound (2)]

Compounds (I), (II) and (III), wherein
R₁ is
an acetyl group,
a benzoyl group,
a methanesulfonyl group,
a p-toluenesulfonyl group,
a benzyl group,
a p-methoxybenzyl group,
a trityl group,
a dimethoxytrityl group,
a monomethoxytrityl group, or
a tert-butyldiphenylsilyl group
(preferably, a tert-butyldiphenylsilyl group),
or a salt thereof.

### [Compound (3)]

Compound (III), wherein
R₃ is
a hydrogen atom,
a phenoxyacetyl group,
an alkyl group having a carbon number of 1 - 5,
an alkenyl group having a carbon number of 2 - 5,
an aryl group having a carbon number of 6 - 14 and optionally having substituent(s),
a methyl group substituted by 1 to 3 aryl groups optionally having substituent(s), a lower aliphatic sulfonyl group optionally having substituent(s) (e.g., methanesulfonyl, trifluoromethanesulfonyl),
an aromatic sulfonyl group optionally having substituent(s) (e.g., p-toluenesulfonyl),
an aliphatic acyl group having a carbon number of 1 - 5 and optionally having substituent(s) (e.g., acetyl), or
an aromatic acyl group optionally having substituent(s) (e.g., phenoxyacetyl, benzoyl)
(preferably, a hydrogen atom, an alkyl group having a carbon number of 1 - 5, a p-toluenesulfonyl group, a methanesulfonyl group, or a trifluoromethanesulfonyl group; more preferably an alkyl group having a carbon number of 1 - 5),
or a salt thereof, and
compound (III), wherein the functional molecular unit substituent for R₃ is a fluorescence□luminescence-labeling molecule or a chemical luminescence-labeling molecule, a functional group having nucleic acid cleavage activity, or an intracellular localization signal peptide or nuclear localization signal peptide, or a salt thereof.

### [Compound (4)]

Compound (I), wherein R₄ is a lower alkylsulfonyl group optionally substituted by a halogen atom (preferably, a trifluoromethanesulfonyl group), or a salt thereof.

### [Compound (5)]

Compound (II), wherein R_{4'} is an alkyl having a carbon number of 1 - 6 - carbonyl group optionally having substituent(s) (preferably, an acetyl group), or a salt thereof.

### [Compound (6)]

Compound (I) or (II), wherein R₅ is a sulfonyl group optionally having substituent(s) (preferably, an aromatic sulfonyl group optionally having substituent(s)), or a salt thereof.

### [Compound (7)]

Compound (I) or (II), wherein R₅ is a phenylsulfonyl group optionally having substituent(s) (preferably, a p-toluenesulfonyl group), or a salt thereof.

### [Compound (8)]

Compound (I), (II) or (II), wherein B is
a 6-aminopurin-9-yl group (i.e., adeninyl),
6-aminopurin-9-yl wherein the amino group is protected by a protecting group for nucleic acid synthesis,
2,6-diaminopurin-9-yl,
a 2-amino-6-chloropurin-9-yl group,
a 2-amino-6-chloropurin-9-yl group wherein the amino group is protected by a protecting group for nucleic acid synthesis,
a 2-amino-6-fluoropurin-9-yl group,
a 2-amino-6-fluoropurin-9-yl group wherein the amino group is protected by a protecting group for nucleic acid synthesis,
a 2-amino-6-bromopurin-9-yl group,
a 2-amino-6-bromopurin-9-yl group wherein the amino group is protected by a protecting group for nucleic acid synthesis,
a 2-amino-6-hydroxypurin-9-yl group (i.e., guaninyl),
a 2-amino-6-hydroxypurin-9-yl group wherein the amino group is protected by a protecting group for nucleic acid synthesis,
a 6-amino-2-methoxypurin-9-yl group,
a 6-amino-2-chloropurin-9-yl group,
a 6-amino-2-fluoropurin-9-yl group,
a 2,6-dimethoxypurin-9-yl group,
a 2,6-dichloropurin-9-yl group, or
a 6-mercaptopurine-9-yl group (preferably 6-aminopurin-9-yl group),
or a salt thereof.

### [Compound (9)]

Compound (I), wherein
R₁ is
an acetyl group,
a benzoyl group,
a methanesulfonyl group,
a p-toluenesulfonyl group,
a benzyl group,
a p-methoxybenzyl group,
a trityl group,
a dimethoxytrityl group,
a monomethoxytrityl group, or
a tert-butyldiphenylsilyl group (preferably, tert-butyldiphenylsilyl group),
R₄ is a lower alkylsulfonyl group optionally substituted by a halogen atom (e.g., a trifluoromethanesulfonyl group),
R₅ is a phenylsulfonyl group optionally having substituent(s) (e.g., a p-toluenesulfonyl group),
B is
a 6-aminopurin-9-yl group (i.e., adeninyl),
6-aminopurin-9-yl wherein the amino group is protected by a protecting group for nucleic acid synthesis,
2,6-diaminopurin-9-yl,
a 2-amino-6-chloropurin-9-yl group,
a 2-amino-6-chloropurin-9-yl group wherein the amino group is protected by a protecting group for nucleic acid synthesis,
a 2-amino-6-fluoropurin-9-yl group,
a 2-amino-6-fluoropurin-9-yl group wherein the amino group is protected by a protecting group for nucleic acid synthesis,
a 2-amino-6-bromopurin-9-yl group,
a 2-amino-6-bromopurin-9-yl group wherein the amino group is protected by a protecting group for nucleic acid synthesis,
a 2-amino-6-hydroxypurin-9-yl group (i.e., guaninyl),
a 2-amino-6-hydroxypurin-9-yl group wherein the amino group is protected by a protecting group for nucleic acid synthesis,
a 6-amino-2-methoxypurin-9-yl group,
a 6-amino-2-chloropurin-9-yl group,
a 6-amino-2-fluoropurin-9-yl group,
a 2,6-dimethoxypurin-9-yl group,
a 2,6-dichloropurin-9-yl group, or
a 6-mercaptopurine-9-yl group (preferably a 6-aminopurin-9-yl group),
or a salt thereof.

### [Compound (10)]

Compound (II), wherein
R₁ is
an acetyl group,
a benzoyl group,
a methanesulfonyl group,
a p-toluenesulfonyl group,
a benzyl group,
a p-methoxybenzyl group,
a trityl group,
a dimethoxytrityl group,
a monomethoxytrityl group, or
a tert-butyldiphenylsilyl group (preferably, a tert-butyldiphenylsilyl group),
R_{4'} is an alkyl having a carbon number of 1 - 6 - carbonyl group optionally having substituent(s) (e.g., an acetyl group),
R₅ is a phenylsulfonyl group optionally having substituent(s) (e.g., a p-toluenesulfonyl group),
B is
a 6-aminopurin-9-yl group (i.e., adeninyl),
a 6-aminopurin-9-yl wherein the amino group is protected by a protecting group for nucleic acid synthesis,
2,6-diaminopurin-9-yl,
a 2-amino-6-chloropurin-9-yl group,
a 2-amino-6-chloropurin-9-yl group wherein the amino group is protected by a protecting group for nucleic acid synthesis,
a 2-amino-6-fluoropurin-9-yl group,
a 2-amino-6-fluoropurin-9-yl group wherein the amino group is protected by a protecting group for nucleic acid synthesis,
a 2-amino-6-bromopurin-9-yl group,
a 2-amino-6-bromopurin-9-yl group wherein the amino group is protected by a protecting group for nucleic acid synthesis,
a 2-amino-6-hydroxypurin-9-yl group (i.e., guaninyl),
a 2-amino-6-hydroxypurin-9-yl group wherein the amino group is protected by a protecting group for nucleic acid synthesis,
a 6-amino-2-methoxypurin-9-yl group,
a 6-amino-2-chloropurin-9-yl group,
a 6-amino-2-fluoropurin-9-yl group,
a 2,6-dimethoxypurin-9-yl group,
a 2,6-dichloropurin-9-yl group, or
a 6-mercaptopurine-9-yl group (preferably 6-aminopurin-9-yl group),
or a salt thereof.

### [Compound (11)]

Compound (III), wherein
R₁ is
an acetyl group,
a benzoyl group,
a methanesulfonyl group,
a p-toluenesulfonyl group,
a benzyl group,
a p-methoxybenzyl group,
a trityl group,
a dimethoxytrityl group,
a monomethoxytrityl group, or
a tert-butyldiphenylsilyl group (preferably, a tert-butyldiphenylsilyl group), R₃ is
a hydrogen atom,
a phenoxyacetyl group,
an alkyl group having a carbon number of 1 - 5,
an alkenyl group having a carbon number of 2 - 5,
an aryl group having a carbon number of 6 - 14 and optionally having substituent(s),
a methyl group substituted by 1 to 3 aryl groups optionally having substituent(s), a lower aliphatic sulfonyl group optionally having substituent(s) (e.g., methanesulfonyl, trifluoromethanesulfonyl),
an aromatic sulfonyl group optionally having substituent(s) (e.g., p-toluenesulfonyl),
an aliphatic acyl group having a carbon number of 1 - 5 and optionally having substituent(s) (e.g., acetyl), or
an aromatic acyl group optionally having substituent(s) (e.g., phenoxyacetyl, benzoyl)
(preferably, a hydrogen atom, an alkyl group having a carbon number of 1 - 5, a p-toluenesulfonyl group, a methanesulfonyl group, or a trifluoromethanesulfonyl group; more preferably an alkyl group having a carbon number of 1 - 5), and compound (III), wherein the functional molecular unit substituent for R₃ is a fluorescence□luminescence-labeling molecule or a chemical luminescence-labeling molecule, a functional group having nucleic acid cleavage activity, or an intracellular localization signal peptide or nuclear localization signal peptide, or a salt thereof.

Among the above-mentioned compounds (1) - (11), the compounds 11', 12', 26', 27', 28', 20' and 21' described in the following Examples, and salts thereof are more preferable.

### [Compound (A-1)]

A compound represented by the formula:

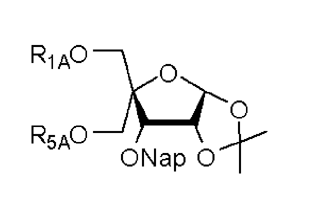

wherein
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
wherein R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5;
R_{5A} is a sulfonyl group optionally having substituent(s) (preferably, a p-toluenesulfonyl group); and
Nap is a 2-naphthylmethyl group;
or a salt thereof.
Of compounds (A-1), a compound wherein
R_{1A} is -Si(R_{8A})(R_{9A})(R_{10A})
wherein R_{8A}, R_{9A} and R_{10A} are the same or different and each is
(i) a lower alkyl group, or
(ii) an aryl group optionally having substituent(s) selected from the group consisting of a lower alkyl group, a lower alkoxy group, a halogen atom and a cyano group (preferably a tert-butyldiphenylsilyl group),
or a salt thereof is preferable.
Of compounds compound (A-1), compound 7' described in the following Example and a salt thereof are more preferable.

### [Compound (A-2)]

A compound represented by the formula:

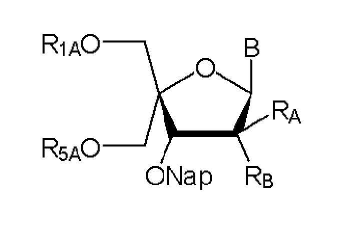

wherein
B is a purine ring group optionally having substituent(s) (preferably, a 6-aminopurin-9-yl group wherein the amino group is optionally protected by a protecting group for nucleic acid synthesis (preferably a benzoyl group);
one of R_{A} and R_{B} is a hydrogen atom, and the other of R_{A} and R_{B} is a hydroxyl group optionally having substituent(s);
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A} wherein R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5;
R_{5A} is a sulfonyl group optionally having substituent(s) (preferably, a p-toluenesulfonyl group); and
Nap is a 2-naphthylmethyl group; and
R_{1A} is not 2-naphthylmethyl,
or a salt thereof.
Of compounds (A-2), a compound wherein
R_{1A} is -Si(R_{8A})(R_{9A})(R_{10A})
wherein R_{8A}, R_{9A} and R_{10A} are the same or different and each is
(i) a lower alkyl group, or
(ii) an aryl group optionally having substituent(s) selected from the group consisting of a lower alkyl group, a lower alkoxy group, a halogen atom and a cyano group (preferably a tert-butyldiphenylsilyl group),
   or a salt thereof is preferable.
Of compounds (A-2), a compound wherein
R_{A} is a hydrogen atom, and
R_{B} is a hydroxyl group optionally having substituent(s),
or a salt thereof is more preferable.
Of compounds (A-2), a compound wherein
R_{A} is a hydrogen atom,
R_{B} is a hydroxyl group optionally having substituent(s) selected from
(1) an acyl group optionally having substituent(s);
(2) a sulfonyl group optionally having substituent(s); and
(3) -NR_{11A}R_{12A}
   wherein R_{11A} and R_{12A} are the same or different and each is
   (i) a hydrogen atom,
   (ii) a hydrocarbon group optionally having substituent(s),
   (iii) a heterocyclic group optionally having substituent(s),
   (iv) an acyl group optionally having substituent(s), or
   (v) a sulfonyl group optionally having substituent(s), or
R_{11A} and R_{12A} form, together with the adjacent nitrogen atom, a cyclic group optionally having substituent(s),
or a salt thereof is more preferable.
Another preferable embodiment of compound (A-2) is a compound wherein
R_{A} is a hydroxyl group optionally having substituent(s), and
R_{B} is a hydrogen atom, or a salt thereof.
Another more preferable embodiment of compound (A-2) is a compound wherein
R_{A} is a hydroxyl group optionally having substituent(s) selected from
(1) an acyl group optionally having substituent(s); and
(2) a sulfonyl group optionally having substituent(s), and
R_{B} is a hydrogen atom, or a salt thereof.
Among compounds (A-2), the compounds 10' - 13' and 23' - 25' described in the following Examples, and salts thereof are particularly preferable.

### [Compound (A-3)]

A compound represented by the formula:

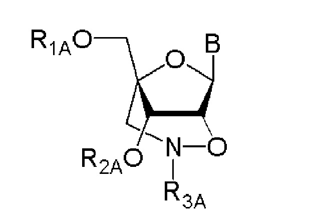

wherein
B is a purine ring group optionally having substituent(s) (preferably, a 6-aminopurin-9-yl group wherein the amino group is optionally protected by a protecting group for nucleic acid synthesis (preferably a benzoyl group);
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
wherein R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5;
R_{2A} is a hydrogen atom or a 2-naphthylmethyl group (preferably a 2-naphthylmethyl group); and
R_{3A} is a hydrogen atom, an alkyl group, an alkenyl group, a cycloalkyl group, an aryl group optionally having substituent(s), an aralkyl group optionally having substituent(s), an acyl group optionally having substituent(s), a sulfonyl group optionally having substituent(s), or a functional molecular unit substituent,
or a salt thereof.
Of compounds (A-3), a compound wherein
R_{1A} is -Si(R_{8A})(R_{9A})(R_{10A})
wherein R_{8A}, R_{9A} and R_{10A} are the same or different and each is
(i) a lower alkyl group, or
(ii) an aryl group optionally having substituent(s) selected from the group consisting of a lower alkyl group, a lower alkoxy group, a halogen atom and a cyano group (preferably a tert-butyldiphenylsilyl group),
or a salt thereof, and a compound wherein
R_{3A} is a hydrogen atom or an alkyl group having a carbon number of 1 - 5,
or a salt thereof, are more preferable.
Among compounds (A-3), the compounds 26', 27', 28', 20' and 21' described in the following Examples, and salts thereof are more preferable, and compound 26' and a salt thereof are still more preferable.

The above-mentioned compound (III) and compound (A-3) are respectively useful as intermediates for synthesizing amidites (e.g., compound 22' described in the following Example or a salt thereof) represented by the formulas:

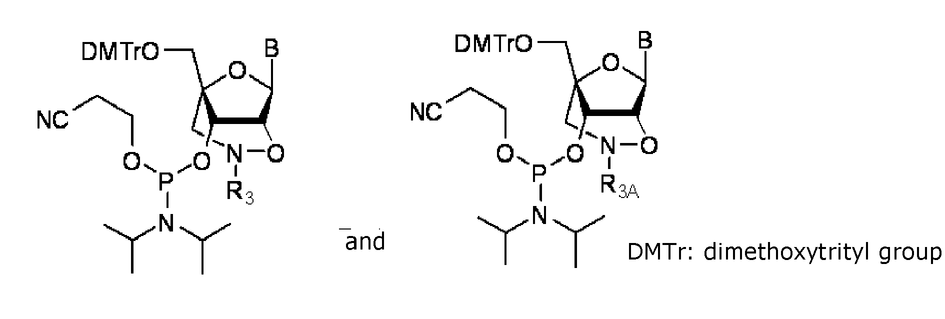

wherein each symbol is as defined above. By introducing the amidite, the "oligonucleotide of the present invention" defined below can be synthesized.

In the above-mentioned compounds (I), (II) and (III) and salts thereof, when B is a 6-aminopurin-9-yl group wherein the amino group is protected by a protecting group for nucleic acid synthesis, the above-mentioned compound (III) and a salt thereof can be produced by, for example, the method shown by the following reaction scheme. When B is a different group, they can be produced similarly, or by a method analogous thereto and the like.
The compounds of the formulas include salts, and examples of such salts include those similar to the salts of the compound of the present invention exemplified above and the like.
The compound obtained in each step can also be used for the next reaction directly as the reaction mixture or as a crude product. It is also possible to isolate the compound from a reaction mixture by a conventional method, and can be purified easily by a separation means such as recrystalliztion, distillation, chromatography and the like.

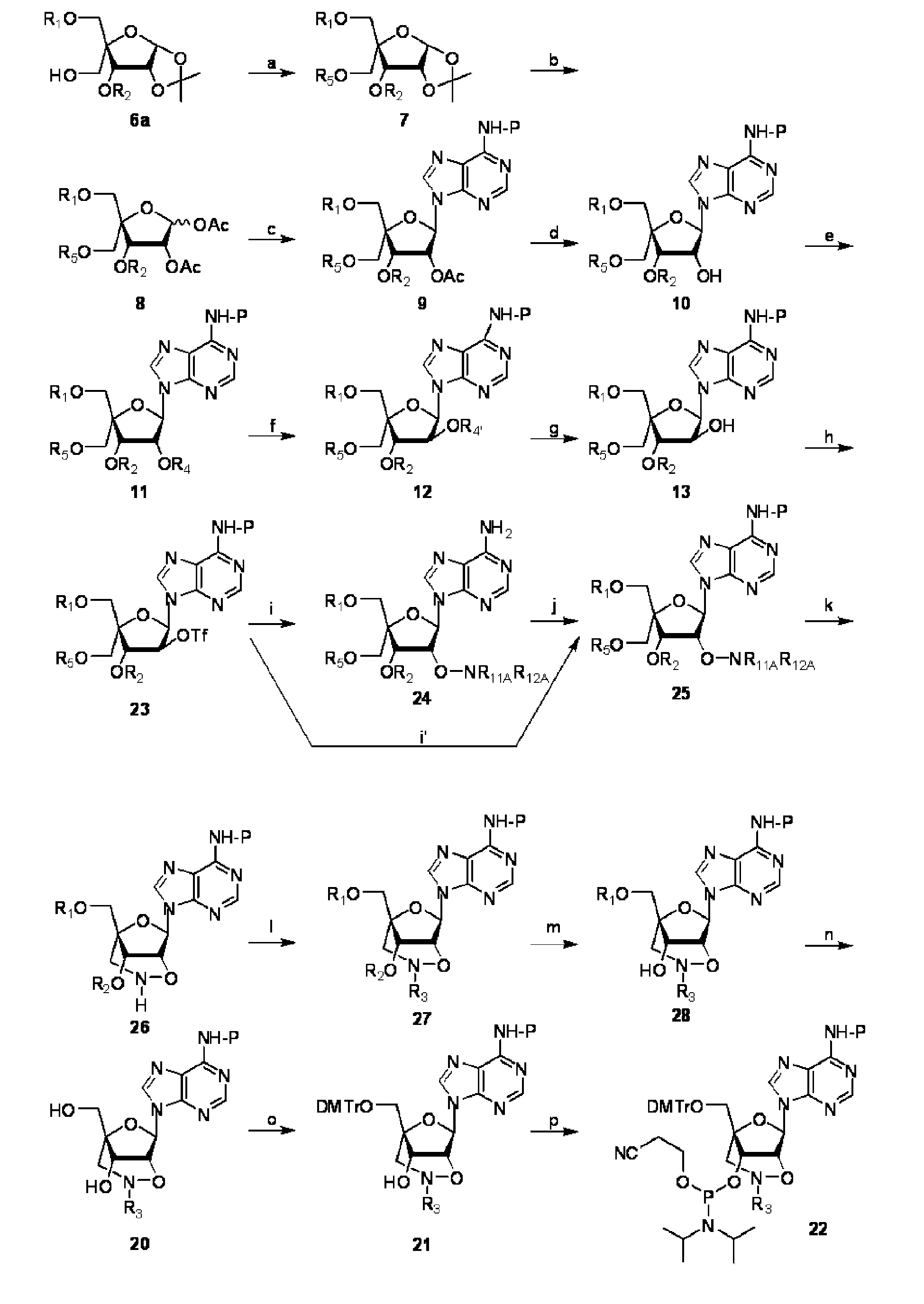

wherein P is the "protecting group" of the aforementioned "amino group protected by a protecting group for nucleic acid synthesis", and other symbols are as defined above.

Compound 6a may be a commercially available product, and can also be produced by the method described in WO2007/090071, or a method analogous thereto.

### step (a)

Compound 7 can be produced by reacting compound 6a with R₅X (X is a halogen atom, and R₅ is as defined above) in a solvent in the presence of a catalyst.
The catalyst is not particularly limited, and a catalyst known *per se* can be used. It is preferably 4-dimethylaminopyridine (DMAP). The amount of the catalyst to be used is not particularly limited and is preferably about 0.5 - 2 equivalents, more preferably about 1 equivalent, relative to 1 equivalent of compound 6a.
As the solvent, a solvent that does not influence the reaction is preferably used, which is preferably an organic solvent, more preferably pyridine.
As R₅X, tosyl chloride is preferably used. The amount of R₅X to be used is not particularly limited and is preferably about 1 - 3 equivalents, more preferably about 2 equivalents relative to 1 equivalent of compound 6a.
The reaction time is generally not less than about 1 hr, preferably about 15 hr.
The reaction temperature is generally about 0 - 200°C, preferably about 60 - 80°C.

### step (b)

Compound 8 can be produced by reacting compound 7 with an acetylation reagent in a solvent.
As the solvent, a solvent that does not influence the reaction is preferably used, which is preferably a carboxylic acid solvent, more preferably acetic acid.
The acetylation reagent is not particularly limited, and an acetylation reagent known *per se* can be used, which is preferably acetic anhydride or acetic acid. The amount of the acetylation reagent to be used is not particularly limited and is preferably about 5 - 10 mL of acetic anhydride and about 10 - 100 mL of acetic acid, more preferably about 8 mL of acetic anhydride and about 30 mL of acetic acid, relative to 1 mmol of compound 7.
The reaction time is generally not less than about 1 hr, preferably about 2 hr.
The reaction temperature is generally about 0°C - room temperature, preferably room temperature.

### step (c)

Compound 9 can be produced by reacting compound 8 with adenine wherein the amino group is protected by a protecting group P for nucleic acid synthesis, in a solvent in the presence of a Lewis acid as a catalyst and using a silylation agent.
As the solvent, a solvent that does not influence the reaction is preferably used, which is preferably an organic solvent, more preferably toluene or acetonitrile, still more preferably acetonitrile.
The Lewis acid as a catalyst is not particularly limited, and a Lewis acid catalyst known *per se* can be used, which is preferably trimethylsilyl trifluoromethanesulfonate (TMSOTf). The amount of the Lewis acid catalyst to be used is not particularly limited and is preferably about 0.5 - 5 equivalents, more preferably about 2 equivalents, relative to 1 equivalent of compound 8.
The silylation agent is not particularly limited, and a silylation agent known *per se* can be used, which is preferably N,O-bis-trimethylsilylacetamide (BSA). The amount of the silylation agent to be used is not particularly limited and is preferably about 2 - 5 equivalents, more preferably about 3 equivalents, relative to 1 equivalent of compound 8.
As the adenine wherein the amino group is protected by a protecting group P for nucleic acid synthesis, N⁶-benzoyladenine can be preferably used. The amount of the adenine wherein the amino group is protected by a protecting group P for nucleic acid synthesis to be used is not particularly limited and is preferably about 1 - 3 equivalents, more preferably about 2 equivalents, relative to 1 equivalent of compound 8.
The reaction time is generally not less than about 1 hr, preferably about 15 hr.
The reaction temperature is generally about 0 - 150°C, preferably 90°C.

### step (d)

Compound 10 can be produced by reacting compound 9 with a deacetylation reagent in a solvent.
As the solvent, a solvent that does not influence the reaction is preferably used, which is preferably an organic solvent, more preferably an alcohol solvent, still more preferably methanol.
The deacetylation reagent is not particularly limited, and a deacetylation reagent known *per se* can be used, which is preferably methanolic ammonia. The amount of the deacetylation reagent to be used is not particularly limited and is preferably about 5 - 20 mL, more preferably about 7.5 mL, relative to 1 mmol of compound 9.
The reaction time is generally not less than about 1 hr, preferably about 15 hr.
The reaction temperature is generally about 0 - 50°C, preferably room temperature.

### step (e)

Compound 11 can be produced by reacting compound 10 with (R₄)₂O(R₄ is as defined above) in the presence of a catalyst in a solvent.
The catalyst is not particularly limited, and a catalyst known *per se* can be used. It is preferably dimethylaminopyridine (DMAP). The amount of the catalyst to be used is not particularly limited and is preferably about 1 - 5 equivalents, more preferably about 3 equivalents, relative to 1 equivalent of compound 10.
As the solvent, a solvent that does not influence the reaction is preferably used, which is preferably an organic solvent, more preferably pyridine.
As (R₄)₂O, trifluoromethanesulfonic anhydride is preferably used. The amount of (R₄)₂O to be used is not particularly limited and is preferably about 1 - 3 equivalents, more preferably about 1.5 equivalents, relative to 1 equivalent of compound 10.
The reaction time is generally not less than about 10 min, preferably about 2 hr.
The reaction temperature is generally about 0 - 150°C, preferably room temperature.

### step (f)

Compound 12 can be produced by inversion reaction of compound 11 in a solvent using (R_{4'})-M (M is an alkali metal salt, and R_{4'} is as defined above).
As the solvent, a solvent that does not influence the reaction is preferably used, which is preferably an organic solvent, more preferably a sulfoxide solvent, still more preferably dimethyl sulfoxide (DMSO).
As (R_{4'})-M, cesium acetate is preferably used. The amount of (R_{4'})-M to be used is not particularly limited and is preferably about 1 - 5 equivalents, more preferably about 3 equivalents, relative to 1 equivalent of compound 11.
The reaction time is generally not less than about 0.5 hr, preferably about 4 hr.
The reaction temperature is generally about 0 - 150°C, preferably room temperature.

### step (g)

Compound 13 can be produced by reacting compound 12 with an oxidant in a solvent.
As the solvent, a solvent that does not influence the reaction is preferably used, and two or more kinds of solvents may be mixed at an appropriate ratio and used. As the solvent, a mixed solvent of an organic solvent and water is preferable, a mixed solvent of halogenated hydrocarbon solvents and water is more preferable and a mixed solvent of dichloromethane and water is still more preferable.
The oxidant is not particularly limited, and an oxidant known *per se* can be used. It is preferably 2,3-dichloro-5,6-dicyano-p-quinone (DDQ). The amount of the oxidant to be used is not particularly limited and is preferably about 1 - 5 equivalents, more preferably about 1.5 equivalents, relative to 1 equivalent of compound 12.
The reaction time is generally not less than about 1 hr, preferably about 6 - 12 hr.
The reaction temperature is generally about 0 - 150°C, preferably room temperature.

### step (h)

Compound 23 can be produced by reacting compound 13 with trifluoromethanesulfonic anhydride in a solvent in the presence of a catalyst.
The catalyst is not particularly limited, and a catalyst known *per se* can be used. It is preferably 1-methylimidazole. The amount of the catalyst to be used is not particularly limited and is preferably about 1 - 5 equivalents, more preferably about 1.1 - 2.0 equivalents, relative to 1 equivalent of compound 13.
As the solvent, a solvent that does not influence the reaction is preferably used, which is preferably an organic solvent, preferably pyridine.
The amount of trifluoromethanesulfonic anhydride to be used is not particularly limited and is preferably about 1 - 5 equivalents, more preferably about 1.1 - 2.0 equivalents, relative to 1 equivalent of compound 13.
The reaction time is generally not less than about 10 min, preferably about 20 min.
The reaction temperature is generally about 0 - 120°C, preferably room temperature.

### step (i)

Compound 24 can be produced by reacting compound 23 with N(OH)R_{11A}R_{12A} (R_{11A} and R_{12A} are as defined above) in the presence of a base in a solvent.
The base is not particularly limited, and a base known *per se* can be used, which is preferably diazabicycloundecene (DBU). The amount of the base to be used is not particularly limited and is preferably about 1 - 5 equivalents, more preferably about 2 equivalents, relative to 1 equivalent of compound 23.
As the solvent, a solvent that does not influence the reaction is preferably used, which is preferably an organic solvent, more preferably a nitrile solvent, and more preferably acetonitrile.
As N(OH)R_{11A}R_{12A}, N-hydroxyphthalimide is preferably used. The amount of N(OH)R_{11A}R_{12A} to be used is not particularly limited and is preferably about 1 - 5 equivalents, more preferably about 2 equivalents, relative to 1 equivalent of compound 23.
The reaction time is generally not less than about 2 hr, preferably about 12 - 48 hr.
The reaction temperature is generally about 0 - 120°C, preferably about 40 - 50°C.

### step (j)

Compound 25 can be produced by reacting compound 24 with a benzoylation reagent in the presence of a base in a solvent.
The base is not particularly limited, and a base known *per se* can be used, which is preferably pyridine. The amount of the base to be used is not particularly limited and is preferably a solvent amount.
As the solvent, an organic solvent is preferable, and pyridine is preferable.
The benzoylation reagent is not particularly limited, and a benzoylation reagent known *per se* can be used, which is preferably benzoyl chloride. The amount of the benzoylation reagent to be used is not particularly limited and is preferably about 2.0 - 5.0 equivalents, more preferably about 2.0 equivalents, relative to 1.0 equivalent of compound 24.
The reaction time is generally not less than about 1 hr, preferably about 5 hr.
The reaction temperature is generally about 0 - 120°C, preferably room temperature.

### step (i')

Compound 25 can be produced by reacting compound 23 with N(OH)R_{11A}R₁₂A (R_{11A} and R_{12A} are as defined above) in the presence of a base in a solvent.
The base is not particularly limited, and a base known *per se* can be used, which is preferably diazabicycloundecene (DBU). The amount of the base to be used is not particularly limited and is preferably about 1 - 5 equivalents, more preferably about 1.1 - 1.8 equivalents, relative to 1 equivalent of compound 23.
As the solvent, a solvent that does not influence the reaction is preferably used, which is preferably an organic solvent, more preferably a nitrile solvent, more preferably acetonitrile.
As N(OH)R_{11A}R_{12A}, N-hydroxyphthalimide is preferably used. The amount of N(OH)R_{11A}R_{12A} to be used is not particularly limited and is preferably about 1 - 5 equivalents, more preferably about 1.1 - 1.8 equivalents, relative to 1 equivalent of compound 23.
The reaction time is generally about 1 - 100 hr, preferably about 10 - 15 hr.
The reaction temperature is generally about 20 - 100°C, preferably about 45 - 55°C.

### step (k)

Compound 26 can be produced by deprotection and cyclization reaction of compound 25 in a solvent using a nucleophile (or nucleophilic reagent) and a catalyst.
As the solvent, an organic solvent is preferable, and pyridine is preferable.
The nucleophile is not particularly limited, and a nucleophile known *per* se can be used, which is preferably hydrazine. The amount of the nucleophile to be used is not particularly limited and is preferably about 1.0 - 2.0 equivalents, more preferably about 1.0 - 1.1 equivalent, relative to 1 equivalent of compound 25.
The catalyst is not particularly limited, and a catalyst known *per se* can be used. It is preferably 1,4-diazabicyclo[2.2.2]octane(DABCO). The amount of the catalyst to be used is not particularly limited and is preferably about 1 - 5 equivalents, more preferably about 1 - 2 equivalents, relative to 1 equivalent of compound 25.
The reaction time of the deprotection is generally not less than about 30 min, preferably about 1 - 3 hr. The reaction time of the cyclization reaction is generally not less than about 3 hr, preferably about 10 - 24 hr.
The reaction temperature is generally about 0 - 120°C, preferably about 100°C.

### step (I)

Compound 27 can be produced by using compound 26 and the method described in patent document 1, or a method analogous thereto.

### steps (m) - (p)

Compound 22 can be produced by using compound 27 by the method described in the following Examples and Reference Examples, or a method analogous thereto.
More specifically, a step of deprotecting compound 27 to give compound 28 (step m), a step of deprotecting compound 28 to give compound 20 (step n), a step of reacting compound 20 with 4,4'-dimethoxytritylchloride (DMTr-Cl) in a solvent (preferably an organic solvent such as pyridine and the like) to give compound 21 (step o), and a step of reacting compound 21 with an amidite reagent such as N,N,N',N'-tetraisopropyl-2-cyanoethylphosphorodiamidite and the like and an activator such as 4,5-dicyanoimidazole and the like in a solvent (preferably an organic solvent such as anhydrous acetonitrile and the like) to give compound 22 (step p) are included.

An NC type nucleoside produced by the method of the present invention can be converted to an NC type nucleotide (hereinafter sometimes to be indicated as a "nucleoside the present invention") by appropriately applying a method generally used in the pertinent field (e.g., the method described in US-B-7217895). The NC type nucleotide can be converted to an oligonucleotide by applying same to a DNA synthesizer and the like together with other known nucleotide (natural nucleotide, artificial nucleotide etc.). The obtained oligonucleotide (sometimes to be indicated as an "oligonucleotide of the present invention" in the present specification) can be purified by using a reversed-phase column. In addition, the production and purity of the purified oligonucleotide can be confirmed by analysis using a reversed-phase HPLC or MALDI-TOF-MS.

The oligonucleotide of the present invention can contain one or more nucleosides of the present invention. In addition, they may be present at one or more sites in the oligonucleotide of the present invention while being separated via one or more natural nucleotides. Moreover, an oligonucleotide containing a necessary number (length) of the nucleotide of the present invention at necessary position(s) introduced thereinto can be synthesized. The total length of the oligonucleotide of the present invention is 2 to 50, preferably 8 to 30, nucleotide units.

Since the oligonucleotide of the present invention contains the nucleotide of the present invention, which is of an NC type, it is not easily degraded by nuclease and can be present in the body for a long time after administration to the body. It can, for example, form a double strand with mRNA with high affinity to inhibit translation into a biocomponent (protein) to be the etiology, and can suppress gene expression due to degradation of the mRNA via endogenous RNase H. In addition, it is considered to inhibit an intracellular growth of an infected virus, or inhibit binding of a nucleotide-binding protein and a target cis-element sequence thereof.

From the above, the oligonucleotide of the present invention is expected to be useful as a "pharmaceutical product that treats a disease by inhibition of gene action" such as antitumor agents and antivirus agents. According to the present invention, therefore, a nucleoside analog which is a production intermediate for an oligonucleotide analog having a stable and superior antisense or antigene activity, or a superior activity as a detection drug for a particular gene or a primer for initiation of amplification, can be provided.

DNA and RNA oligonucleotide analogs (oligonucleotide analogs) obtained by modifying an NC type purine nucleoside, which is one of the nucleotides obtained by applying the method of the present invention, are useful as various physiological or biological active substances, materials of pharmaceutical products, functional materials of double strand oligonucleotides for RNA interference method, decoy method and the like, functional materials of DNA chip, molecular beacon and the like targeting a single strand nucleic acid such as cDNA and the like, functional materials for use in various antisense methods (including ribozyme, DNAzyme), antigene method, and gene homologous recombinant method, materials for high sensitivity analysis of trace biological organic components by combination with fluorescence or luminescence substances, developmental materials of reagents for researches such as gene function analysis elucidation and the like.

The nucleoside analogs, nucleotide analogs and oligonucleotide analogs of the present invention can be added with conventionally-used auxiliary, for example, a buffering agent and/or a stabilizer and the like to give a preparation for parenteral administration. In addition, as a topical preparation, ointment, cream, liquid, plaster and the like can be prepared by adding conventionally-used carriers for medicaments.

While the present invention is explained in detail in the following by referring to Examples and Reference Examples, which are not to be construed as limitative of the present invention.

### Examples

The abbreviations in the Examples and Reference Examples mean as described below.
LC: liquid chromatography
MS: mass spectrometry
ESI: electrospray method
NMR: nuclear magnetic resonance spectrum
Hz: hertz
J: coupling constant
m: multiplet
q: quartet
t: triplet
d: doublet
s: singlet
br: broad
Me: methyl group
Ac: acetyl group
Bz: benzoyl group
Nap: 2-naphthylmethyl group
Ts: p-toluenesulfonyl group
Tf: trifluoromethanesulfonyl group
TBDPS: tert-butyldiphenylsilyl group
DMTr: dimethoxytrityl group
%: mol percent
room temperature: temperature of generally from about 0°C to 35°C

### silica gel column chromatography

Elution was performed under observation by TLC (thin layer chromatography), unless particularly indicated.
TLC plate: 60F254 manufactured by Merck
eluent: hexane/ethyl acetate solvent
detection: UV detector
silica gel for column chromatography: silica gel 60 (70-230 mesh) manufactured by Merck

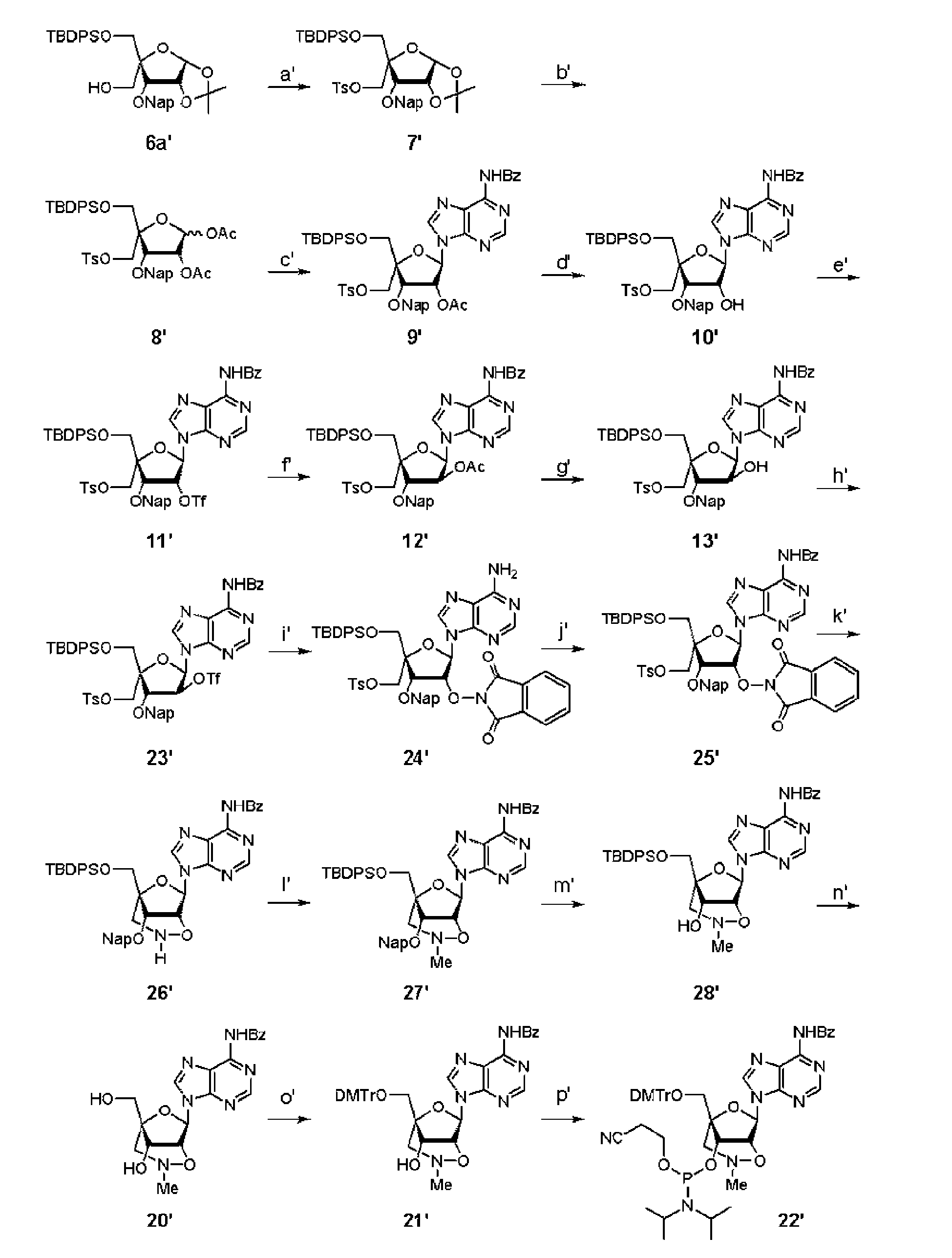

### Example 1: synthesis of p-toluenesulfonylated compound (step a')

Using compound 6a' obtained in the same manner as in Example 3 of WO2007/090071 as a starting material, compound 6a' (15 mmol, 9.0 g), tosyl chloride (30 mmol, 5.7 g) and 4-dimethylaminopyridine (15 mmol, 1.8 g) were dissolved in pyridine (100 mL), and the reaction mixture was stirred at 80°C for 15 hr. The reaction was quenched by adding ice to the reaction mixture, and the reaction mixture was concentrated under reduced pressure. The obtained syrup-like residue was diluted with ethyl acetate, and the organic layer was washed with water (twice) and saturated brine. The combined aqueous layer was extracted with ethyl acetate, and the organic layers were combined and dried over sodium sulfate. Sodium sulfate was filtered off, the filtrate was concentrated and the obtained residue was purified by moderate-pressure silica gel column chromatography (hexane/ethyl acetate, 15→35%) to give compound 7' (p-toluenesulfonylated compound, 10.4 g, 92%) as a white foam.

### Compound 6a'

¹H NMR (CDCl₃, 300 MHz) δ 7.85-7.78 (m, 4H), 7.61-7.49 (m, 7H), 7.41-7.28 (m, 6H), 5.81 (d, 1H, J = 3.6 Hz), 4.97 (d, 1H, J = 11.9 Hz), 4.71 (dd, 1H, overlapped), 4.68 (d, 1H, overlapped), 4.48 (d, 1H, J = 5.3 Hz), 3.87 (m, 2H), 3.78 (d, 1H, J = 11.0 Hz), 3.69 (d, 1H, J = 11.0 Hz), 2.42 (dd, 1H, J = 6.0, 7.5 Hz, exchangeable with D₂O), 1.67, 1.38 (each s, each 3H), 0.92 (s, 9H).

### Compound 7'

¹H NMR (CDCl₃, 300 MHz) δ 7.85-7.69 (m, 6H), 7.57-7.17 (m, 15H), 5.69 (d, 1H, J = 3.6 Hz), 4.85 (d, 1H, J = 12.1 Hz), 4.67 (d, 1H, J = 12.1 Hz), 4.58 (dd, 1H, J = 3.6, 4.9 Hz), 4.50 (d, 1H, J = 10.6 Hz), 4.38 (d, 1H, J = 10.6 Hz), 4.36 (d, 1H, J = 4.9 Hz), 2.36 (s, 3H), 1.36, 1.30 (each s, each 3H), 0.90 (s, 9H).

### Example 2: synthesis of glycosylated compound (steps b', c')

Compound 7' (13.7 mmol, 10.3 g) and acetic anhydride (7.9 mL) were dissolved in acetic acid (31.7 mL), conc. sulfuric acid (15.8 µL) was added, and the reaction mixture was stirred at room temperature for 2 hr. To the reaction mixture was added ice-cooled about 1N aqueous sodium hydroxide solution (about 20 mL), and the reaction mixture was vigorously stirred for several minutes. The reaction mixture was diluted with ethyl acetate, and the organic layer was washed with 0.5N aqueous sodium hydroxide solution, water and saturated brine in this order. The aqueous layer was collected, and extracted again with ethyl acetate. The combined organic layer was neutralized with sodium bicarbonate and dried with sodium sulfate. Sodium bicarbonate and sodium sulfate were filtered off, and the filtrate was washed with water and brine in this order to remove the remaining salt. The organic layer was dried over sodium sulfate, and sodium sulfate was filtered off. The filtrate was concentrated and the obtained residue was co-evaporated 4 times with toluene. The residue was dried under reduced pressure, and the obtained crude product (white foam, compound 8') was directly used for the next reaction.
Compound 8' (crude diacetyl form) was dissolved in acetonitrile (80 mL), N⁶-benzoyladenine (27.4 mmol, 6.55 g), N,O-bis(trimethylsilyl)acetamide (41.1 mmol, 10.0 mL) and molecular sieves 4A (12.0 g) were added, and the mixture was refluxed for 20 min. To the reaction mixture was added trimethylsilyl trifluoromethanesulfonate (27.4 mmol, 5.0 mL) at once, and the mixture was refluxed for 15 hr (oil bath temperature: 90 - 100°C). The reaction mixture was cooled to 0°C, and the insoluble material was filtered off through a celite pad. To the filtrate was added saturated aqueous sodium hydrogen carbonate, and the mixture was vigorously stirred for 10 min. The mixture was extracted twice with ethyl acetate, and the combined organic layer was washed with water and saturated brine in this order. The combined aqueous layer was extracted again with ethyl acetate, and the combined organic layer was dried over sodium sulfate. Sodium sulfate was filtered off, and the filtrate was concentrated. The obtained residue was purified by moderate-pressure silica gel column chromatography (hexane/ethyl acetate, 60→75→90%) to give compound 9' (glycosylated form, 12.5 g, 94% by 2 steps) as a white foam.
¹H NMR (CDCl₃, 300 MHz) δ 8.92 (br s, 1H, exchangeable with D₂O), 8.54 (s, 1H), 8.01 (d, 2H, overlapped), 7.99 (s, 1H), 7.87-7.80 (m, 3H), 7.71-7.51 (m, 12H), 7.43-7.24 (m, 7H), 7.12 (d, 2H, J = 7.9 Hz), 6.08 (d, 1H, J = 5.1 Hz), 6.00 (dd, 1H, J = 5.1, 5.6 Hz), 4.87 (d, 5.6 Hz), 4.76 (d, 1H, J = 11.5 Hz), 4.67 (d, 1H, J = 11.5 Hz), 4.46 (d, 1H, J = 10.6 Hz), 4.33 (d, 1H, J = 10.6 Hz), 3.86 (d, 1H, J = 11.0 Hz), 3.74 (d, 1H, J = 11.0 Hz), 2.32 (s, 3H), 2.04 (s, 3H), 0.95 (s, 9H).

### Example 3: synthesis of acetyl-deprotected compound (step d')

Compound 9' (12.8 mmol, 12.5 g) was dissolved in methanol (192 mL), ammonia (96 mL as about 7M methanol solution) was added, and the reaction mixture was kept at room temperature for 2 hr. The ammonia and solvent were evaporated under reduced pressure, and the obtained residue was purified by moderate-pressure silica gel column chromatography (hexane/ethyl acetate, 70→90→100%) to give compound 10' (acetyl-deprotected form, 10.1 g, 85%) as a white foam.
¹H NMR (CDCl₃, 300 MHz) δ 8.92 (br s, 1H, exchangeable with D₂O) 8.55 (s, 1H), 8.00 (d, 2H, J = 7.0 Hz), 7.92 (s, 1H), 7.87-7.80 (m, 3H), 7.76-7.70 (m, 3H), 7.61-7.31 (m, 15H), 7.23 (d, 1H, J = 7.6 Hz), 7.15 (d, 2H, J = 8.1 Hz), 5.82 (d, 1H, J = 4.9 Hz), 4.90 (d, 1H, J = 11.6 Hz), 4.83 (d, 1H, J = 11.6 Hz), 4.80 (ddd, 1H, overlapped), 4.62 (d, 1H, J = 5.9 Hz), 4.46 (d, 1H, J = 10.5 Hz), 4.41 (d, 1H, J = 10.5 Hz), 3.80 (d, 1H, J = 6.8 Hz, exchangeable with D₂O) 3.76 (d, 1H, J = 10.8 Hz), 3.71 (d, 1H, J = 10.8 Hz), 2.33 (s, 3H), 0.95 (s, 9H).

### Example 4: synthesis of arabino adenosine derivative (steps e', f')

Compound 10' (1.07 mmol, 1.00 g) and 4-dimethylaminopyridine (3.22 mmol, 393 mg) were dissolved in pyridine (7.0 mL), and trifluoromethanesulfonic anhydride (1.61 mmol, 270 µL) was added dropwise. The reaction mixture was stirred at room temperature for 2 hr, ice was added to the reaction mixture to quench the reaction. The obtained solution was partitioned between ethyl acetate and water, the organic layer was washed with half-saturated brine and saturated brine in this order. The aqueous layers were combined, and extracted again with ethyl acetate. The combined organic layer was dried over sodium sulfate. Sodium sulfate was filtered off, the filtrate was concentrated and the obtained residue was co-evaporated 3 times with toluene. The residue was dried under reduced pressure, and the obtained crude product (yellow foam, compound 11') was directly used for the next reaction.
Compound 11' (crude, sulfonyl form) was dissolved in dimethyl sulfoxide (5.0 mL), cesium acetate (2.14 mmol, 410 mg) was added, and the reaction mixture was stirred at room temperature for 2 hr. Since LC-MS analysis confirmed residual 10 - 20% sulfonyl form (compound 11'), cesium acetate (1.07 mmol, 205 mg) was added, and the mixture was further stirred at room temperature for 1.5 hr. The reaction mixture was poured into water, and the mixture was extracted with ethyl acetate. The separated organic layer was washed with water and saturated brine in this order. The aqueous layers were combined, extracted again with ethyl acetate, and the combined organic layer was dried over sodium sulfate. Sodium sulfate was filtered off, and the filtrate was concentrated. The obtained residue was purified by moderate-pressure silica gel column chromatography (hexane/ethyl acetate, 40→60→80%) to give compound 12' (arabino adenosine derivative, 520 mg, 50% by 2 steps) as a yellow foam.
¹H NMR (CDCl₃, 300 MHz) δ 8.95 (br s, 1H, exchangeable with D₂O) 8.61 (s, 1H), 8.02 (d, 2H, J = 7.0 Hz), 7.85 (s, 1H), 7.83-7.73 (m, 7H), 7.56-7.15 (m, 17H), 6.46 (d, 1H, J = 5.3 Hz), 5.49 (dd, 1H, J = 5.3, 4.1 Hz), 4.88 (d, 1H, J = 12.1 Hz), 4.74 (d, 1H, J = 12.1 Hz), 4.65 (d, 1H, J = 4.1 Hz), 4.50 (s, 2H), 3.98 (d, 1H, J = 10.5 Hz), 3.70 (d, 1H, J = 10.5 Hz), 2.28 (s, 3H), 1.44, (s, 3H), 0.88 (s, 9H).

### Example 5: synthesis of 2-naphthylmethyl-deprotected compound (step g')

Compound 12' (0.51 mmol, 500 mg) was dissolved in methylene chloride (5.0 mL), and water (0.5 mL) was added to give a two-layer mixture. To the mixture was added 2,3-dichloro-5,6-dicyano-p-quinone (1.54 mmol, 350 mg), and the resulting dark green reaction mixture was vigorously stirred at room temperature for 64 hr. The reaction mixture was diluted with ethyl acetate, and washed with saturated aqueous sodium thiosulfate solution, saturated aqueous sodium hydrogen carbonate and saturated brine in this order. The aqueous layers were combined, extracted again with ethyl acetate, and the combined organic layer was dried over sodium sulfate. Sodium sulfate was filtered off, and the filtrate was concentrated. The obtained residue was purified by moderate-pressure silica gel column chromatography (hexane/ethyl acetate, 50→70→85%) to give compound 13' (2-naphthylmethyl-deprotected compound, 398 mg, 93%) as a pale-brown foam.
¹H NMR (CDCl₃, 300 MHz) δ 8.99 (br s, 1H, exchangeable with D₂O) 8.56 (s, 1H), 8.02 (d, 1H, J = 7.1 Hz), 7.89 (s, 1H), 7.87 (d, 2H, J = 8.3 Hz), 7.62-7.21 (m, 15H), 6.45 (d, 1H, J = 6.4 Hz), 5.44 (dd, 1H, J = 6.4, 6.5 Hz), 4.98 (m, 1H), 4.50 (d, 1H, J = 10.4 Hz), 4.45 (d, 1H, J = 10.4 Hz), 4.00 (d, 1H, 10.6 Hz), 3.95 (d, 1H, J = 10.6 Hz), 3.24 (br s, 1H, exchangeable with D₂O) 2.43 (s, 3H), 1.60 (s, 3H), 1.00 (s, 9H).

### Example 6: synthesis of N⁶-dibenzoyl compound (steps h', i', j')

Compound 13' (3.0 mmol, 2.8 g) was dissolved in pyridine (15 mL), 1-methylimidazole (3.6 mmol, 0.29 mL) and trifluoromethanesulfonic anhydride (3.6 mmol, 0.61 mL) were added, and the mixture was stirred at room temperature for 30 min. The reaction mixture was poured into ice water to quench the reaction, and the mixture was extracted twice with ethyl acetate. The combined organic layer was washed with water (twice) and saturated brine in this order, and dried over sodium sulfate. Sodium sulfate was filtered off and the filtrate was concentrated. The obtained residue was co-evaporated with toluene and directly used for the next reaction.
The crude sulfonyl form (compound 23') obtained in the previous step was dissolved in acetonitrile (15 mL), N-hydroxyphthalimide (12.0 mmol, 2.0 g) and diazabicycloundecene (12.0 mmol, 1.2 mL) were added, and the reaction mixture was refluxed for 16 hr. Water was added to quench the reaction, and the mixture was partitioned between ethyl acetate and water. The organic layer was washed with water and brine in this order, dried over sodium sulfate, and sodium sulfate was filtered off. The obtained residue was crudely purified by moderate-pressure silica gel column chromatography (hexane/ethyl acetate, 80→100%) to give a crude compound 24' (phthalimidoxy-substituted compound).
The crude phthalimidoxylated form (compound 24') obtained in the previous step was dissolved in pyridine, benzoyl chloride (6.0 mmol, 0.7 mL) was added, and the reaction mixture was stirred at room temperature for 22 hr. To the reaction mixture was added ice to quench the reaction, and the mixture was partitioned between ethyl acetate and water. The organic layer was washed with water and brine in this order, dried over sodium sulfate, and sodium sulfate was filtered off. The obtained residue was purified by moderate-pressure silica gel column chromatography (hexane/ethyl acetate, 40→60%) to give compound 25' (N⁶-dibenzoyl form, 0.83 g, 23% by 3 steps) in an as a white foam.
¹H NMR (CDCl₃, 300 MHz) δ 8.11 (s, 1H), 7.87 (s, 1H), 7.86-7.74 (m, 15H), 7.52-7.15 (m, 21H), 6.63 (d, 1H, J = 3.4 Hz), 5.60 (dd, 1H, J = 3.4, 6.3 Hz), 5.32 (d, 1H, J = 11.5 Hz), 4.96 (d, 1H, J = 6.3 Hz), 4.93 (d, 1H, J = 11.5 Hz), 4.70 (d, 1H, J = 11.2 Hz), 4.37 (d, 1H, J = 11.2 Hz), 3.93 (d, 1H, J = 11.2 Hz), 3.75 (d, 1H, J = 11.2 Hz), 2.31 (s, 3H), 0.83 (s, 9H).

### Example 7: (step k')

Compound 25' (0.34 mmol, 400 mg) was dissolved in pyridine (3.4 mL), hydrazine (0.41 mmol, 18 µL) was added, and the reaction mixture was stirred at room temperature for 2 hr.
To the reaction mixture was added 1,4-diazabicyclo[2.2.2]octane (0.68 mmol, 76 mg), and the reaction mixture was stirred at 70°C for 18 hr. The reaction mixture was poured into water, and extracted twice with ethyl acetate. The combined organic layer was washed with brine, dried over sodium sulfate, and sodium sulfate was filtered off. The obtained residue was co-evaporated with toluene, and purified by moderate-pressure silica gel column chromatography (hexane/ethyl acetate, 70→85→100%) to give compound 26' (BNA ^{Nc}-adenosine derivative, 108 mg, 41% by 2 steps), as a white foam.
¹H NMR (CDCl₃, 300 MHz) δ 9.00 (br s, 1H, exchangeable with D₂O) 8.74, 8.36 (each s, each 1H), 8.02 (d, 2H, J = 7.0 Hz), 7.82-7.74 (m, 4H), 7.65-7.26 (m, 16H), 6.72 (s, 1H), 6.13 (br s, 1H, exchangeable with D₂O) 4.99 (d, 1H, J = 2.3 Hz), 4.92 (d, 1H, J = 11.5 Hz), 4.76 (d, 1H, J = 11.5 Hz), 4.47 (d, 1H, J = 2.3 Hz), 3.92 (d, 1H, J = 11.9 Hz), 3.77, 3.77 (each d, each 1H, overlapped), 2.70 (d, 1H, J = 12.6 Hz), 1.01 (s, 9H).

### Example 8: (step I')

Compound 26' (0.11 mmol, 90 mg) was dissolved in 10% acetic acid (methanol solution, 2.2 mL), formalin (0.14 mmol, 11 µL) was added, and the reaction mixture was stirred at room temperature for 20 min.
To the reaction mixture was added a borane-picoline complex (0.22 mmol, 23 mg), and the reaction mixture was stirred at room temperature for 6 hr. To the reaction mixture was added 1N aqueous hydrochloric acid solution, and the obtained mixture was partitioned between ethyl acetate and 0.1N aqueous hydrochloric acid solution. The organic layer was washed with saturated aqueous sodium hydrogen carbonate and brine in this order, dried over sodium sulfate, and sodium sulfate was filtered off. The obtained residue co-evaporated with toluene, and purified by moderate-pressure silica gel column chromatography (hexane/ethyl acetate, 50→70→100%) to give compound 27' (BNA^{NC}-adenosine derivative, 86 mg, 99%) as a white foam.
¹H NMR (CDCl₃, 300 MHz) δ 8.94 (br s, 1H, exchangeable with D₂O) 8.75, 8.34 (each s, each 1H), 8.02 (d, 2H, J = 7.2 Hz), 7.83-7.75 (m, 4H), 7.66-7.26 (m, 16H), 6.84 (s, 1H), 4.97 (d, 1H, J = 2.5 Hz), 4.90 (d, 1H, J = 11.5 Hz), 4.73 (d, 1H, J = 11.5 Hz), 4.47 (d, 1H, J = 2.5 Hz), 3.93 (d, 1H, J = 11.7 Hz), 3.78 (d, 1H, J = 11.7 Hz), 3.06, 3.80 (each d, each 1H, overlapped), 2.82 (s, 3H), 1.01 (s, 9H).

### Example 9: (step m')

Compound 27' (0.24 mmol, 190 mg) was dissolved in dichloromethane (4.8 mL), water (0.48 mL) and 2,3-dichloro-5,6-dicyano-p-benzoquinone (0.48 mmol, 109 mg) were added, and the obtained solution was stirred at room temperature for 6 hr. The solution after stirring was partitioned between ethyl acetate and water, and the organic layer was washed with saturated aqueous sodium thiosulfate solution, saturated aqueous sodium hydrogen carbonate and saturated brine in this order, and dried over sodium sulfate. Sodium sulfate was filtered off, and the filtrate was concentrated. The obtained residue was purified by moderate-pressure column chromatography (hexane/ethyl acetate), and the obtained compound 28' was subjected to the next step (n').

### Example 10: (step n')

Compound 28' was dissolved in tetrahydrofuran (4 mL), triethylamine (0.11 mmol, 15 µL) and triethylaminetrihydrofluoride (0.21 mmol, 34 µL) were added, and the reaction mixture was stirred at room temperature for 4 hr. The obtained reaction mixture was concentrated, and the residue was purified by moderate-pressure silica gel column chromatography (ethyl acetate/methanol) to give compound 20' (46 mg, yield from compound 27' 47%) as a white foam.
compound 20':¹H NMR(300MHz,DMSO-d₆)δ 2.72(s,3H), 2.86(s,2H), 3.62(m,2H), 4.18(dd,J=5.5,3.2,1H), 4.52(d,J=3.0,1H), 5.12(t,J=5.9,1H), 5.47(d,J=5.5,1H), 6.67(s,1H), 7.48-7.72(m,3H), 8.01-8.09(m,2H), 8.60(s,1H), 8.75(s,1H), 11.22(br s,1H).

### Example 11: (step o')

Compound 20' (300 mg, 0.72 mmol) was dried by co-evaporating with dry pyridine, and dissolved in pyridine (4 ml). 4,4'-Dimethoxytrityl chloride (271 mg, 0.8 mmol) was added thereto, and the mixture was stirred at room temperature for 12 hr. To the reaction mixture was added saturated aqueous sodium hydrogen carbonate solution (10 mL), and the aqueous layer was extracted with ethyl acetate (25 mL). The obtained organic layer was washed with saturated aqueous sodium hydrogen carbonate solution (10 mL), and dried over anhydrous sodium sulfate, and concentrated. The residue was purified by silica gel column chromatography (hexane-ethyl acetate) to give compound 21' (416 mg, 0.58 mmol).
¹H NMR (300MHz,CDCl₃) δ 2.78 (s,3H), 2.82-3.11 (m,3H), 3.38 (s,2H), 3.77 (s,6H), 4.39 (br.s,1H), 4.67 (d,J=2.8Hz,1H), 6.75-6.89 (m,5H), 7.15-7.64 (m,12H), 8.00 (d,2H), 8.33 (s,1H), 8.79 (s,1H), 9.17 (br.s,1H).

### Reference Example 1:(step p')

Compound 21' (410 mg, 0.57 mmol) was dried by co-evaporating (twice) with anhydrous acetonitrile, and dissolved in anhydrous acetonitrile (2.5 ml). N,N,N',N'-Tetraisopropyl-2-cyanoethylphosphorodiamidite (200 µL, 0.63 mmol) and 4,5-dicyanoimidazole (DCI, 71 mg, 0.6 mmol) were successively added thereto. The mixture was stirred at room temperature for 2 hr, and the reaction mixture was diluted with ethyl acetate (20 mL). Saturated aqueous sodium hydrogen carbonate solution (10 mL) was added thereto to quench the reaction. The organic layer was washed with saturated aqueous sodium hydrogen carbonate solution (10 mL), dried over anhydrous sodium sulfate, and concentrated. The residue was purified by silica gel column chromatography (Diol-silica gel (manufactured by Fuji Silysia, hexane-acetone) to give the objective BNA^{Nc}-adenosine monomer (compound 22': 480 mg, 0.52 mmol).
¹H NMR (300MHz,CDCl₃) δ 0.92-1.22 (m,12H), 2.26-2.48 (m,2H), 2.76, 2.78 (2s,3H), 2.82-2.95 (m,2H), 3.27-3.64 (m,4H), 3.78-3.79 (m,6H), 4.095,4.48 (2dd,1H,J1=8.03Hz,3.12Hz,J2=7.18Hz,3.02Hz), 4.67,4.80 (2d,1H,J=2.83Hz,J=2.83Hz), 6.52,6.55 (2s,1H) 6.80-6.83 (m,2H) , 7.21-7.36 (m,9H), 7.43-7.46 (m,2H), 8.35, 8.42 (2s,1H), 8.84 (s,1H), 9.05 (br.s,1H).
³¹P NMR (121MHz) δ 149.08, 149.36

### Industrial Applicability

According to the present invention, a method of producing a purine NC type nucleoside efficiently and conveniently in a high yield without a protecting group conversion step and an intermediate compound therefor are provided.

This application is based on a patent application No. 2009-148994 filed in Japan, the contents of which are incorporated in full herein. In addition, the patent documents and non-patent documents cited in the present specification are hereby incorporated in their entireties by reference, to the extent that they have been disclosed in the present specification.

## Claims

1. A method of producing a compound represented by the formula (II): 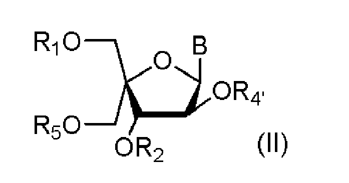 or a salt thereof, comprising inverting a compound represented by the formula (I): 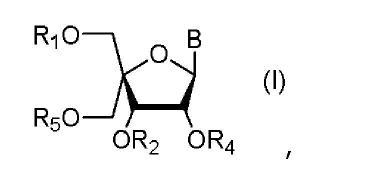 in each formula,
B is a purine ring group optionally having substituent(s);
R₁ is a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R₆)R₇
(R₆ and R₇ are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R₂ is a 2-naphthylmethyl group;
R₄ is an alkylsulfonyl group optionally substituted by a halogen atom;
R_{4'} is an acyl group optionally having substituent(s); and
R₅ is a sulfonyl group optionally having substituent(s).

2. A method of producing a compound represented by the formula (III): 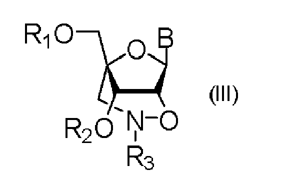 or a salt thereof, comprising a step of producing a compound represented by the formula (II): 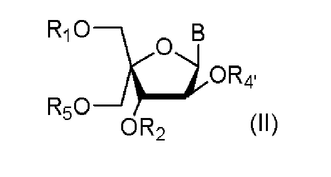 or a salt thereof, by inverting a compound represented by the formula (I): 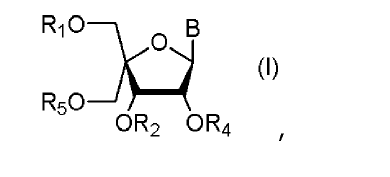 in each formula,
B is a purine ring group optionally having substituent(s);
R₁ is a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R₆)R₇
(R₆ and R₇ are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R₂ is a 2-naphthylmethyl group;
R₃ is a hydrogen atom, an alkyl group, an alkenyl group, a cycloalkyl group, an aryl group optionally having substituent(s), an aralkyl group optionally having substituent(s), an acyl group optionally having substituent(s), a sulfonyl group optionally having substituent(s), or a functional molecular unit substituent;
R₄ is an alkylsulfonyl group optionally substituted by a halogen atom;
R_{4'} is an acyl group optionally having substituent(s); and
R₅ is a sulfonyl group optionally having substituent(s).

3. The method according to claim 1 or 2, wherein B is a purine ring group optionally having substituent(s), which is bonded at the 9-position of the purine ring.

4. The method according to claim 1 or 2, wherein B is a purin-9-yl group optionally having substituent(s) selected from the following group a
[group a: a hydroxyl group,
a hydroxyl group protected by a protecting group for nucleic acid synthesis,
an alkoxy group having a carbon number of 1 - 5,
a mercapto group,
a mercapto group protected by a protecting group for nucleic acid synthesis,
an alkylthio group having a carbon number of 1 - 5,
an amino group,
an amino group protected by a protecting group for nucleic acid synthesis,
an amino group substituted by an alkyl group having a carbon number of 1 - 5, an alkyl group having a carbon number of 1 - 5 and
a halogen atom].

5. The method according to claim 1 or 2, wherein B is
a 6-aminopurin-9-yl group,
a 6-aminopurin-9-yl group wherein the amino group is protected by a protecting group for nucleic acid synthesis,
a 2,6-diaminopurin-9-yl group,
a 2-amino-6-chloropurin-9-yl group,
a 2-amino-6-chloropurin-9-yl group wherein the amino group is protected by a protecting group for nucleic acid synthesis,
a 2-amino-6-fluoropurin-9-yl group,
a 2-amino-6-fluoropurin-9-yl group wherein the amino group is protected by a protecting group for nucleic acid synthesis,
a 2-amino-6-bromopurin-9-yl group,
a 2-amino-6-bromopurin-9-yl group wherein the amino group is protected by a protecting group for nucleic acid synthesis,
a 2-amino-6-hydroxypurin-9-yl group,
a 2-amino-6-hydroxypurin-9-yl group wherein the amino group is protected by a protecting group for nucleic acid synthesis,
a 6-amino-2-methoxypurin-9-yl group,
a 6-amino-2-chloropurin-9-yl group,
a 6-amino-2-fluoropurin-9-yl group,
a 2,6-dimethoxypurin-9-yl group,
a 2,6-dichloropurin-9-yl group or
a 6-mercaptopurin-9-yl group.

6. The method according to claim 1 or 2, wherein B is a 6-aminopurin-9-yl group.

7. The method according to claim 1 or 2, wherein R₁ is
(1) an aliphatic acyl group optionally having substituent(s),
(2) an aromatic acyl group optionally having substituent(s),
(3) an aliphatic sulfonyl group optionally having substituent(s),
(4) an aromatic sulfonyl group optionally having substituent(s),
(5) a methyl group substituted by 1 to 3 aryl groups optionally having 1 to 3 substituents, or
(6) -Si(R₈)(R₉)(R₁₀)
wherein R₈, R₉ and R₁₀ are the same or different and each is
(i) a lower alkyl group or
(ii) an aryl group optionally having substituent(s) selected from the group consisting of a lower alkyl group, a lower alkoxy group, a halogen atom and a cyano group.

8. The method according to claim 1 or 2, wherein R₁ is
an acetyl group,
a benzoyl group,
a methanesulfonyl group,
a p-toluenesulfonyl group,
a benzyl group,
a p-methoxybenzyl group,
a trityl group,
a dimethoxytrityl group,
a monomethoxytrityl group or
a tert-butyldiphenylsilyl group.

9. The method according to claim 1 or 2, wherein R₁ is a tert-butyldiphenylsilyl group.

10. The method according to claim 2, wherein R₃ is
a hydrogen atom,
a phenoxyacetyl group,
an alkyl group having a carbon number of 1 - 5,
an alkenyl group having a carbon number of 2 - 5,
an aryl group having a carbon number of 6 - 14 and optionally having substituent(s),
a methyl group substituted by 1 to 3 aryl groups optionally having substituent(s), a lower aliphatic sulfonyl group optionally having substituent(s),
an aromatic sulfonyl group optionally having substituent(s),
an aliphatic acyl group having a carbon number of 1 - 5 and optionally having substituent(s), or
an aromatic acyl group optionally having substituent(s).

11. The method according to claim 2, wherein R₃ is
a hydrogen atom,
an alkyl group having a carbon number of 1 - 5,
a p-toluenesulfonyl group,
a methanesulfonyl group, or
a trifluoromethanesulfonyl group.

12. The method according to claim 2, wherein R₃ is an alkyl group having a carbon number of 1 - 5.

13. The method according to claim 2, wherein the functional molecular unit substituent for R₃ is a fluorescence□luminescence-labeling molecule or chemical luminescence-labeling molecule, a functional group having nucleic acid cleavage activity, or an intracellular or nuclear localization signal peptide.

14. The method according to claim 1 or 2, wherein R₄ is a lower alkylsulfonyl group optionally substituted by a halogen atom.

15. The method according to claim 1 or 2, wherein R₄ is a trifluoromethanesulfonyl group.

16. The method according to claim 1 or 2, wherein R_{4'} is an alkyl having a carbon number of 1 - 6 - carbonyl group optionally having substituent(s).

17. The method according to claim 1 or 2, wherein R_{4'} is an acetyl group.

18. The method according to claim 1 or 2, wherein R₅ is an aromatic sulfonyl group optionally having substituent(s).

19. The method according to claim 1 or 2, wherein R₅ is a phenylsulfonyl group optionally having substituent(s).

20. The method according to claim 1 or 2, wherein R₅ is a p-toluenesulfonyl group.

21. The method according to any one of claims 1 to 20, wherein the reaction is performed in a solvent in the presence of an organic acid salt of an alkali metal.

22. The method according to claim 21, wherein the solvent is a sulfoxide solvent.

23. The method according to claim 21, wherein the solvent is dimethyl sulfoxide.

24. The method according to claim 21, wherein the organic acid salt of alkali metal is alkali metal acetate.

25. The method according to claim 21, wherein the organic acid salt of alkali metal is cesium acetate.

26. The method according to any one of claims 21 to 25, wherein the reaction temperature is room temperature.

27. A compound represented by the formula: 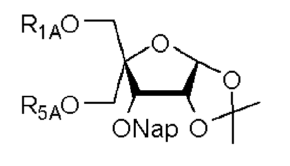 wherein
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
(R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R_{5A} is a sulfonyl group optionally having substituent(s); and
Nap is a 2-naphthylmethyl group;
or a salt thereof.

28. The compound according to claim 27, wherein R_{1A} is -Si(R_{8A})(R_{9A})(R_{10A})
wherein R_{8A}, R_{9A} and R_{10A} are the same or different and each is
(i) a lower alkyl group, or
(ii) an aryl group optionally having substituent(s) selected from the group consisting of a lower alkyl group, a lower alkoxy group, a halogen atom and a cyano group,
or a salt thereof.

29. A compound represented by the formula: 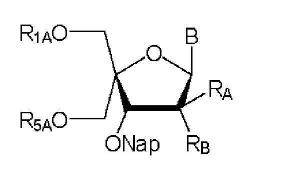 wherein
B is a purine ring group optionally having substituent(s);
one of R_{A} and R_{B} is a hydrogen atom, and the other of R_{A} and R_{B} is a hydroxyl group optionally having substituent(s);
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
(R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R_{5A} is a sulfonyl group optionally having substituent(s); and
Nap is a 2-naphthylmethyl group; and
R_{1A} is not 2-naphthylmethyl, or a salt thereof.

30. The compound according to claim 29, wherein R_{1A} is -Si(R_{8A})(R_{9A})(R_{10A})
wherein R_{8A}, R_{9A} and R_{10A} are the same or different and each is
(i) a lower alkyl group, or
(ii) an aryl group optionally having substituent(s) selected from the group consisting of a lower alkyl group, a lower alkoxy group, a halogen atom and a cyano group, or a salt thereof.

31. The compound according to claim 29, wherein R_{A} is a hydrogen atom, and
R_{B} is a hydroxyl group optionally having substituent(s), or a salt thereof.

32. The compound according to claim 29, wherein R_{A} is a hydrogen atom, and
R_{B} is a hydroxyl group optionally having substituent(s) selected from
(1) an acyl group optionally having substituent(s);
(2) a sulfonyl group optionally having substituent(s); and
(3) -NR_{11A}R_{12A}
(R_{11A} and R_{12A} are the same or different and each is
(i) a hydrogen atom,
(ii) a hydrocarbon group optionally having substituent(s),
(iii) a heterocyclic group optionally having substituent(s),
(iv) an acyl group optionally having substituent(s), or
(v) a sulfonyl group optionally having substituent(s), or
R_{11A} and R_{12A} form, together with the adjacent nitrogen atom, a cyclic group optionally having substituent(s)),
or a salt thereof.

33. The compound according to claim 29, wherein R_{A} is a hydroxyl group optionally having substituent(s), and
R_{B} is a hydrogen atom, or a salt thereof.

34. The compound according to claim 29, wherein R_{A} is a hydroxyl group optionally having substituent(s) selected from
(1) an acyl group optionally having substituent(s); and
(2) a sulfonyl group optionally having substituent(s), and
R_{B} is a hydrogen atom, or a salt thereof.

35. A compound represented by the formula: 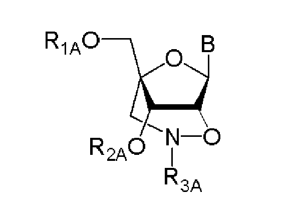 wherein
B is a purine ring group optionally having substituent(s);
R_{1A} is a hydrogen atom, a hydroxyl-protecting group for nucleic acid synthesis, an alkyl group, an alkenyl group, a cycloalkyl group, a nonaromatic heterocyclic group, an aryl group optionally having substituent(s), an aralkyl group optionally having substituent(s), an acyl group optionally having substituent(s), a sulfonyl group optionally having substituent(s), a silyl group optionally having substituent(s), a phosphate group, a phosphate group protected by a protecting group for nucleic acid synthesis, or -P(R_{6A})R_{7A}
(R_{6A} and R_{7A} are the same or different and each is a hydroxyl group, a hydroxyl group protected by a protecting group for nucleic acid synthesis, a mercapto group, a mercapto group protected by a protecting group for nucleic acid synthesis, an amino group, an alkoxy group having a carbon number of 1 - 5, an alkylthio group having a carbon number of 1 - 5, a cyanoalkoxy group having a carbon number of 1 - 6, or an amino group substituted by an alkyl group having a carbon number of 1 - 5);
R_{2A} is a hydrogen atom or a 2-naphthylmethyl group; and
R_{3A} is a hydrogen atom, an alkyl group, an alkenyl group, a cycloalkyl group, an aryl group optionally having substituent(s), an aralkyl group optionally having substituent(s), an acyl group optionally having substituent(s), a sulfonyl group optionally having substituent(s), or a functional molecular unit substituent;
or a salt thereof.

36. The compound according to claim 35, wherein R_{1A} is -Si(R_{8A})(R_{9A})(R_{10A}) (R_{8A}, R_{9A} and R_{10A} are the same or different and each is
(i) a lower alkyl group, or
(ii) an aryl group optionally having substituent(s) selected from the group consisting of a lower alkyl group, a lower alkoxy group, a halogen atom and a cyano group),
or a salt thereof.

37. The compound according to claim 35, wherein R_{3A} is a hydrogen atom or an alkyl group having a carbon number of 1 - 5, or a salt thereof.

38. The compound according to any one of claims 29 to 37, wherein B is a 6-aminopurin-9-yl group wherein the amino group is optionally protected by a protecting group for nucleic acid synthesis, or a salt thereof.

39. The compound according to any one of claims 27 to 34, wherein R_{5A} is a p-toluenesulfonyl group, or a salt thereof.
